# EUROPÄISCHE PATENTSCHRIFT

(11) **EP 0 519 873 B1**
(45) Veröffentlichungstag und Bekanntmachung des Hinweises auf die Patenterteilung: **27.12.1996**
(21) Anmeldenummer: 92810464.5
(22) Anmeldetag: 16.06.1992
(51) Int. Cl.: A61F 2/38

(54) **Gelenkprothese, insbesondere Kniegelenkprothese**
Joint prosthesis, particularly for the knee
Prothèse d'articulation, en particulier du genou

(30) Priorität: 17.06.1991 CH 1795/91; 17.06.1991 CH 1796/91; 19.12.1991 CH 3783/91
(43) Veröffentlichungstag der Anmeldung: 23.12.1992
(60) Teilanmeldung: 95109878.9
(73) Patentinhaber: Bähler, André, CH-8032 Zürich (CH)
(72) Erfinder: Bähler, André, CH-8032 Zürich (CH)
(74) Vertreter: Riederer, Conrad A., Dr.

(56) Entgegenhaltungen:
- EP-A- 0 186 471
- EP-A- 0 336 774
- EP-A- 0 381 352
- WO-A-89/06947
- WO-A-92/08424
- DE-U- 9 110 504
- FR-A- 2 290 883
- FR-A- 2 568 467
- FR-A- 2 663 536
- GB-A- 2 061 730
- GB-A- 2 223 950
- US-A- 4 207 627

## Beschreibung

Die Erfindung betrifft eine Gelenkprothese, insbesondere Kniegelenkprothese, mit mindestens einem ersten Prothesenteil, das einen Verankerungsabschnitt und mindestens einen Drehgelenkabschnitt aufweist, einem zweiten Prothesenteil, das einen Verankerungsabschnitt und eine Gleitfläche aufweist, und einem Zwischenteil, welches um einen Drehzapfen verschwenkbar ist und mindestens einen Drehgelenkabschnitt zur Zusammenarbeit mit dem entsprechenden Drehgelenkabschnitt des ersten Prothesenteils aufweist.

Ein bis heute nur unvollkommen gelöstes Problem der Endoprothetik ist die unveränderte Aufrechterhaltung eines stabilen Knochen/Implantat-Verbundes über lange Jahre, im Idealfall bis zum Tode des Prothesenträgers. Es sind vor allem zwei Faktoren, welche dagegen wirken. Es sind dies zum einen die an der Knochen/Implantat-Grenze, dem sogenannten Interface, auftretenden, nach Betrag und Richtung wechselnden Kräfte, insbesondere Scherkräfte, und zum andern knochenzerstörende, biologische Gewebereaktionen auf Fremdkörpermaterial, insbesondere auf Abriebpartikel von den Prothesenteilen. Soll also die Langlebigkeit einer Gelenkprothese erhöht werden, so sind durch geeignete Massnahmen die am Interface angreifenden Wechselkräfte klein zu halten und der an den Gleitflächen auftretende Verschleiss zu minimieren. Dies kann auf verschiedene Weise geschehen, doch sind nicht alle Methoden gleichermassen gangbar. Es genügt nämlich nicht, nur die genannten technischen Aspekte zu berücksichtigen, sondern es muss auch auf die anatomischen und physiologischen Verhältnisse Rücksicht genommen werden. Insbesondere bei Gelenken mit einer verhältnismässig komplizierten Kinematik, wie z.B. beim Kniegelenk, kann es zu schwer zu lösenden Zielkonflikten kommen.

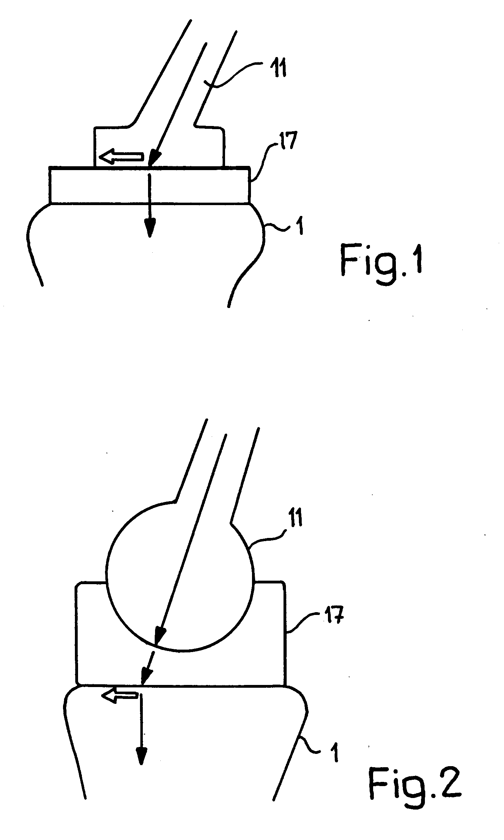

Bekanntlich ist bei Gleitlagern der Verschleiss umso geringer, je kleiner der Flächendruck der aufeinander reibenden Gleitpartner ist. Der Flächendruck, und damit auch der resultierende Verschleiss, ist klein, wenn die eigentliche Kontaktfläche der beiden Gleitpartner gross ist. Die Kontaktfläche ist dann gross, wenn die Gleitflächen möglichst gross und kongruent ausgebildet sind. Typische Beispiele hierfür sind das Schlittengelenk (Fig. 1) und das Scharniergelenk (Fig. 2). Obwohl das Schlittengelenk im Prinzip als ein Scharniergelenk mit unendlichem Radius betrachtet werden kann, bestehen aber ausser dem gemeinsamen Merkmal des minimierten Verschleisses grundsätzliche Unterschiede. Das Schlittengelenk ist translativ frei beweglich, besitzt aber keine Drehachse. Umgekehrt besitzt das Scharniergelenk eine Drehachse, ist aber translativ gefangen. Dies hat unterschiedliches Verhalten gegenüber von aussen einwirkenden Kräften zur Folge.

Beim Schlittengelenk bewirkt z.B. eine schräg von oben kommende Kraft eine Verschiebung des Gelenkkopfes auf der Gleitfläche in Richtung der horizontalen Komponente, ohne dass der untere Gelenkteil und damit dessen Interface von dieser Horizontalkraft selbst betroffen würde.

Beim Scharniergelenk hingegen geht die gleiche Kraft durch das Gelenk hindurch, ohne im Gelenk eine Bewegung auszulösen, induziert jedoch am Interface des unteren Gelenkteils eine Horizontalkomponente und damit eine unerwünschte Scherkraft, die zudem ständig ihre Richtung wechselt, wenn der obere Gelenkteil hin und her pendelt. Vereinfacht gesagt wird mit dem Scharniergelenk der Vorteil der Drehbeweglichkeit mit dem Nachteil der translativen Immobilität und der Scherkraftbelastung am Interface erkauft. Nun sind Körpergelenke selten reine Scharnier- oder Schlittengelenke, vielmehr findet sich, wie z.B. beim Kniegelenk, eine Kombination davon, indem der Drehbewegung eine gleichzeitige Translation überlagert ist.

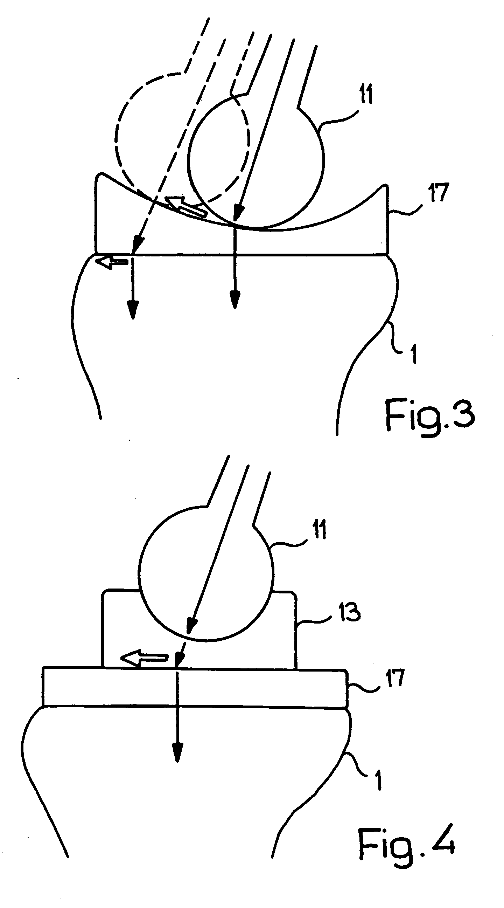

Um diese Kombination von Bewegungen zu ermöglichen, muss das Scharniergelenk "geöffnet" werden, d.h. die Kongruenz der Gleitflächen muss herabgesetzt werden (Fig. 3). Dies bedeutet aber, kleine Kontaktflächen mit entsprechend grossem Flächendruck, was erhöhten Verschleiss zur Folge hat. Der Verschleiss wird zusätzlich durch die repetitive Translation des Gelenkkopfes auf der Gleitfläche erhöht, was infolge des fokussierten Flächendrucks zu einem Walkprozess mit besonders schneller Materialermüdung des untenliegenden Gelenkteils führt. Zudem schützt die Inkongruenz nicht unbedingt vor Scherkräften am Interface. Ueberhöhte Gleitflächen an der Peripherie wirken sich kräftemässig analog aus, wie ein Scharniergelenk. Diese Nachteile, erhöhter Verschleiss und Scherkräfte am Interface mit den damit verbundenen negativen Auswirkungen, sind bei allen Prothesen dieser Bauart wohlbekannt.

Eine andere bekannte Lösung ist die Kombination von Schlitten- und Scharniergelenk auf zwei Ebenen mit Hilfe eines Zwischenteils (Fig. 4). Der Drehbewegung des Scharniergelenks wird gewissermassen die Translationsbewegung des Schlittengelenks unterlagert. Bei diesem Prinzip bleibt die Kongruenz der Gelenkflächen voll erhalten, und der Verschleiss wird minimiert. Zudem werden die durch das Scharniergelenk durchgehenden Kräfte nicht auf das Interface übertragen, sondern im Zwischenteil in Translationsbewegung umgesetzt. Es existieren auch Prothesen dieser Bauart und sind als sogenannte Meniskus-Knie bekannt.

Eine derartige Prothese wird z.B. in der US-A-4,085,466 beschrieben. Diese ist bikompartimantär ausgebildet und weist für jedes Kompartiment einen Femurteil, einen Tibiateil und einen dazwischen angeordneten Meniskusteil auf, der die Form einer runden Scheibe besitzt. Oben besitzt der Meniskusteil eine konkave Ausnehmung, deren Radius dem Radius des Femurteils entspricht. Unten besitzt der Meniskusteil eine runde Aussparung, die nach oben konisch erweitert ist. In diese Aussparung erstreckt sich ein pilzförmiger Vorsprung des Tibiateils. Der Durchmesser des Einlasses der Aussparung ist etwas kleiner als der Durchmesser des Kopfes des pilzförmigen Vorsprungs, so dass beim Aufsetzen des aus Kunststoff oder elastischem Material bestehenden Meniskusteils auf den Tibiateil ein Einschnappen des Vorsprungs in die Ausnehmung erfolgt. Nach dem Einschnappen ist eine Gleitbewegung des Meniskusteils in allen Richtungen bis zum Anschlagen des Vorsprungs an der Seitenwandung der Aussparung möglich. Nachteilig ist dabei, dass nur eine kurze Anterior-Posterior-Bewegung möglich ist. Auch ist die Rotationsbewegung stark beschränkt, weil das Gelenk bikompartimentär ist und daher der pilzförmige Vorsprung des einen Kompartiments, die Drehung um den pilzförmigen Vorsprung des anderen Kompartiments stark begrenzt. Besonders nachteilig ist das Anschlagen des Meniskusteils am Ende jeder Translationsbewegung, weil dadurch am Interface unerwünschte Scherkräfte erzeugt werden. Schliesslich sind auch seitliche Translationsbewegungen unerwünscht.

In der EP-B-0 021 421 werden zwei verschiedene Kniegelenkprothesen beschrieben, welche auch als "Oxford-Knie" und "New Jersey-Knie" bezeichnet werden. Das Oxford-Knie weist zwei Femurteile, zwei Zwischenteile und zwei Tibiateile auf. Die Femurteile bestehen aus je einem sphärischen Segment, das einen Verankerungsabschnitt zur Verankerung im Femur aufweist. Die Zwischenteile sind runde Scheiben mit einer sphärischen Vertiefung zur Lagerung des entsprechenden Femurteils. Die Tibiateile besitzen einen Verankerungsabschnitt zur Verankerung in der Tibia und ein Plateau, auf welchem das Zwischenteil gleiten kann. Als nachteilig erweist sich dabei, dass bei einer Flexion von 90 Grad und mehr das Zwischenteil über das Plateau teilweise hinausgeschoben und eventuell ganz disloziert werden kann. Die gleiche Gefahr besteht auch, wenn aus irgendwelchen Gründen die Spannung der nach der Operation verbliebenen Gelenkbänder nachlässt und sich somit die Femurteile von den Zwischenteilen abheben lassen. In solchen Fällen ist stets eine erneute Operation des Kniegelenks notwendig.

Das New Jersey-Knie begegnet der beschriebenen Gefahr des Hinausschiebens durch spezielle Vorrichtungen. Am Tibiateil, das für beide Zwischenteile vorgesehen ist, befinden sich zwei schwalbenschwanzartige, bogenförmige Nuten, durch welche die Zwischenteile zwangsgeführt werden. Die Bogen sind jeweils gegen das Zentrum zu gerichtet, so dass das Zwischenteil beim Beugen des Knies nicht unkontrolliert rückwärtsgleiten und über das Plateau hinausgeschoben werden kann, sondern am dort noch verbliebenen zentralen Knochenzapfen anschlägt. Die Schwalbenschwanzform andererseits verhindert ein Luxieren des Zwischenteils beim Nachlassen der Bänderspannung. Der Nachteil dieser Ausführung ist die Zwangsführung der Zwischenteile auf eine vorbestimmte Bahn, welche nur für eine einzige, singuläre Gelenkstellung eine Kongruenz der Gelenkflächen zwischen Femurteil und Zwischenteil zulässt, für jede andere Gelenkstellung jedoch zu einer verschleissfördernden Inkongruenz der Gelenkflächen führt. Eine Inkongruenz entsteht, weil die am Femur fest verankerten Femurteile stets im gleichen Abstand zueinander stehen und eine gemeinsame Drehachse haben, die Zwischenteile jedoch beim Vor- und Rückwärtsgleiten auf der bogenförmigen Bahn sich seitlich einander nähern oder voneinander entfernen, wobei ausserdem die Scharniergelenkachsen stetig ihre Stellung zueinander ändern. Dasselbe, nur im umgekehrten Sinne, geschieht auch bei Rotationsbewegungen um eine Achse senkrecht zum Plateau. Der ständig wechselnde Grad der dieser Prothese innewohnenden Inkongruenz der Scharniergelenkflächen kompromittiert natürlich das verschleissminimierende Prinzip des Meniskusknie in grundsätzlicher Weise.

In der EP-B-0 018 364 wird eine Kniegelenkprothese beschrieben, auch "rotating platform" genannt, welche ein Femurteil, ein Tibiateil und ein Zwischenteil aufweist. Das Femurteil besitzt einen Verankerungsabschnitt zur Befestigung im Femur und zwei Kondylen. Das Tibiateil besitzt einen Verankerungsabschnitt zur Befestigung in der Tibia und eine zentrale Bohrung zur Aufnahme eines Drehzapfens des Zwischenteils. Das Zwischenteil besitzt einen in die zentrale Bohrung des Tibiateils passenden Drehzapfen sowie zwei konkave Gleitlager zur Lagerung der Kondylen des Femurteils. Dadurch weist diese Prothese den Vorteil der verschleissmindernden Kongruenz der Gleitflächen des Scharniergelenks auf und zudem wird die Luxation des Zwischenteils wegen des Drehzapfens verhindert.

Im Gegensatz jedoch zu den vorhergehend diskutierten Beispielen besteht wegen des Drehzapfens keine Möglichkeit der translativen Bewegung des Femurteils relativ zum Tibiateil, sondern nur die Möglichkeit der Rotation um die Achse des Drehzapfens. Es handelt sich hier also um ein klassisches Scharniergelenk mit der zusätzlichen Freiheit der Rotation um eine senkrecht zu diesem Gelenk stehende Achse. Demzufolge gehen auch schräg von oben kommende Kräfte, wie sie z.B. durch den Muskelzug oder die Gewichtsbelastung beim Aufsetzen des Fusses entstehen, durch das Gelenk hindurch auf das Interface und induzieren dort die unerwünschten Scherkräfte. Auch in diesem Fall wird das Prinzip des Meniskusknie kompromittiert, indem die verschleissmindernde Kongruenz der Gelenkflächen mit dem Verlust der verankerungsschonenden Translationsmöglichkeit und der damit verbundenen natürlichen Kniekinematik erkauft wird.

Die WO-A-9208424, welche eine ältere nachveröffentlichte Anmeldung darstellt, zeigt eine Kniegelenkprothese mit einem ersten und einem zweiten Prothesenteil und einem dazwischen liegenden Meniskus- oder Zwischenteil, welches ein Langloch aufweist. In diesem Langloch befindet sich ein kurzes Führungsstück, das mit einer im zweiten Prothesenteil eingeschraubten Ansatzschraube drehbar befestigt ist. Dies ermöglicht dem Zwischenteil sowohl eine Rotationsbewegung als auch eine Anterior-Posterior-Bewegung. Die Rotationsbewegung wird durch Anschläge begrenzt. Die Anterior-Posterior-Bewegung, also Translationsbewegung, wird durch die Länge des Langlochs begrenzt. Nachteilig bei dieser Prothese ist die relativ kurze Translationsbewegung, weil wegen Platzmangel das Langloch notgedrungen kurz gehalten werden muss. Das kurze Langloch bedingt relativ kleine Führungsflächen am Führungsstück und dementsprechend hohe spezifische Flächenpressungen. Dies wiederum führt zu hohem Verschleiss, wobei auch ein Verkanten und Verklemmen möglich ist. Als nachteilig erweist sich aber auch das ständige Anschlagen des Zwischenteils am Ende jeder Translationsbewegung, und die dadurch an der Implantat/Knochen-Grenze auftretenden wechselnden Kräfte, wie dies bereits einleitend geschildert wurde.

Es ist daher Aufgabe der vorliegenden Erfindung, die beschriebenen Nachteile bekannter Prothesen zu vermeiden. Insbesondere soll eine Prothese geschaffen werden, welche nicht nur Gelenkflächenkongruenz und Translationsmöglichkeit des sogenannten Meniskusknies aufweist, sondern diese auch bei Rotationsbewegungen aufrecht erhält und weitgehende Luxationssicherheit des Zwischenteils gewährleistet sowie einen annähernd normalen, physiologischen Bewegungsablauf ermöglicht.

Diese Aufgabe wird gemäss der Erfindung durch eine Gelenkprothese gemäss Anspruch 1 gelöst. Die erfindungsgemässe Prothese ermöglicht einen Bewegungsablauf, der weitgehend dem normalen physiologischen Bewegungsablauf entspricht. So verlagern sich bei der Flexion die Kondylen von ventral nach dorsal. Gleichzeitig kann aber auch eine Drehbewegung stattfinden. Sowohl bei der Flexion als auch bei der Extension sind daher ähnliche Verhältnisse gegeben wie beim natürlichen Knie. Die Schwenkbewegung und die Translationsbewegung von anterior nach posterior und umgekehrt werden nach dem Einbau der Prothese grundsätzlich durch die Bänder des Kniegelenks begrenzt. Auch ist in jeder Gelenkstellung die Kongruenz der Scharniergelenkflächen des ersten Prothesenteils und des Zwischenteils gewährleistet, da die auf dem Zwischenteil angeordneten Gelenkflächen zueinander unbeweglich sind. Von oben kommende Kräfte gelangen nicht zum Interface, sondern werden im Zwischenteil in Translationsbewegung umgesetzt. Schliesslich ist auch die Gefahr der gefürchteten Dislokation des Zwischenteils bei der Flexion gebannt.

Bei einem vorteilhaften Ausführungsbeispiel der Erfindung ist der Drehzapfen am Kupplungsorgan angeordnet und im zweiten Prothesenteil schwenkbar gelagert. Dies ergibt eine besonders einfache Konstruktion. Der Drehzapfen kann dabei in einer Bohrung gelagert sein, die sich in einen Verankerungsabschnitt erstreckt. Der Drehzapfen kann somit relativ lang sein, so dass keine Gefahr besteht, dass er nach dem Einbau der Prothese aus der Bohrung gehoben wird. Es sind auch keine speziellen Mittel zur axialen Sicherung des Drehzapfens notwendig.

Ein anderes Ausführungsbeispiel der Erfindung sieht vor, dass der Drehzapfen am zweiten Prothesenteil angeordnet ist und dass das Kupplungsorgan auf dem Drehzapfen verschwenkbar gelagert ist. Diese Konstruktion hat den Vorteil, dass keine zentrale Ausnehmung am Knochen für die Aufnahme eines Verankerungsabschnitts notwendig ist. Die Verankerungsabschnitte können daher am zweiten Prothesenteil ohne Rücksicht auf den Drehzapfen angeordnet sein, z.B. in einer peripheren Lage. Die Führungsbahn verläuft zweckmässigerweise in Richtung anterior-posterior, also mindestens angenähert in physiologischer Gleitrichtung. Bei einem Kniegelenk kann somit die Führungsbahn in etwa sagittaler Richtung angeordnet werden. Dabei ist es vorteilhaft, die Führungsbahn in einem Bogen mit medialem Krümmungsradius verlaufen zu lassen. Die Konstruktion ist vorzugsweise so gestaltet, dass die Beweglichkeit des Zwischenteils relativ zum Kupplungsorgan auf Bewegungen in der Gleitbahnebene eingeschränkt ist. So ist es möglich, der Führungsbahn einen schwalbenschwanz-, T-förmigen, polygonalen oder kreisförmigen Querschnitt zu geben. Durch diese Ausgestaltung wird verhindert, dass der Führungsteil die Führungsbahn verlässt, wenn aus irgendwelchen Gründen die Spannung der nach der Operation verbliebenen Gelenkbänder nachlässt. Die Führungsbahn ist zweckmässigerweise praktisch mittig im Zwischenteil angeordnet.

Die Drehgelenkabschnitte können verschiedenartig ausgebildet sein, um eine Drehbewegung zu ermöglichen. Eine vorteilhafte Ausführungsform sieht vor, dass der Drehgelenkabschnitt des ersten Prothesenteils durch zwei Kondylen gebildet ist, und dass der Drehgelenkabschnitt des Zwischenteils durch zwei entsprechende Lagerflächen zur Zusammenarbeit mit den Kondylen des ersten Prothesenteils gebildet ist. Dies ergibt eine Prothese, die dem natürlichen Gelenkaufbau, wie er beispielsweise beim Kniegelenk zu finden ist, sehr nahe kommt.

Eine andere Ausführungsform, die sich beispielsweise für ein monokompartimentes Gelenk eignet, sieht vor, dass der Drehgelenkabschnitt des ersten Prothesenteils nur durch eine Kondyle gebildet ist, und dass der Drehgelenkabschnitt des Zwischenteils ebenfalls nur durch eine Lagerfläche zur Zusammenarbeit mit der Kondyle des ersten Prothesenteils gebildet ist.

Vorteilhafterweise ist die jeweilige Kondyle des ersten Prothesenteils konvex und die zugehörige Lagerfläche des Zwischenteils entsprechend konkav. Dies entspricht dem natürlichen Gelenkaufbau. Es wäre aber auch möglich, wo die anatomische Struktur dies nahelegt, die jeweilige Kondyle des ersten Prothesenteils als konkave Lägerfläche und die entsprechende Lagerfläche des Zwischenteils als konvexe Kondyle zu gestalten.

Weiter ist es möglich, dass die jeweilige Lagerfläche des Zwischenteils ein in eine Ausnehmung desselben eingesetztes auswechselbares Element ist. Dies ermöglicht es, intraoperativ verschieden hohe Lagerelemente einzupassen, um einseitige Bandlaxizitäten auszugleichen oder Achsfehlstellungen zu korrigieren. Es ist auch möglich, Zwischenteil und Lagerelemente aus verschiedenen Materialien zu fertigen. So könnten beispielsweise das Zwischenteil aus Metall und die Lagerelemente aus Kunststoff bestehen.

Wenn auswechselbare Lagerelemente vorgesehen werden, so ist es möglich, die Ausnehmung im Zwischenteil entweder durchgehend oder nicht durchgehend zu gestalten. Wenn die Ausnehmung durchgehend ist, liegt das Lagerelement auf der Gleitfläche des zweiten Prothesenteils auf. Dies kann von Vorteil sein, wenn das Lagerelement aus einem Material besteht, das gute Gleiteigenschaften auf dem Material der Gleitfläche aufweist. Zweckmässigerweise wird dann das Zwischenteil z.B. durch das Kupplungsorgan in einem Abstand von der Gleitfläche gehalten. Bei der Ausbildung der Prothese als Kniegelenkprothese ist es vorteilhaft, wenn das zweite Prothesenteil und das Zwischenteil dorsal eine Ausnehmung für das hintere Kreuzband aufweisen. Die erfindungsgemässe Ausbildung der Prothese lässt solche Ausnehmungen zu. Dies hat den Vorteil, dass die für den Bewegungsablauf des Kniegelenks wichtige Funktion des hinteren Kreuzbandes aufrechterhalten wird.

Es ist auch möglich, die Führungsbahn auf dem Kupplungsorgan anzuordnen, welches auf dem zweiten Prothesenteil verschwenkbar gelagert ist, um dem Zwischenteil zusätzlich zur Translationsbewegung auch eine Schwenkbewegung relativ zum zweiten Prothesenteil zu ermöglichen. Dies ermöglicht einen Bewegungsablauf, der weitgehend dem normalen physiologischen Bewegungsablauf entspricht. So verlagern sich bei der Flexion die Kondylen des ersten Prothesenteils, also bei einer Kniegelenkprothese jene des Femurteils, von anterior nach posterior. Gleichzeitig kann aber auch eine Drehbewegung um eine Achse senkrecht zum Plateau des zweiten Prothesenteils stattfinden. Sowohl bei der Flexion als auch bei der Extension sind daher ähnliche Verhältnisse gegeben wie beim natürlichen Knie. Auch ist in jeder Gelenkstellung die Kongruenz der Scharniergelenkflächen des ersten Prothesenteils und des Zwischenteils gewährleistet, da die auf dem Zwischenteil angeordneten Gelenkflächen zueinander unbeweglich sind. Von oben kommende Kräfte erzeugen keine Scherkräfte am Interface, sondern werden durch den Zwischenteil in Translationsbewegung umgesetzt. Schliesslich ist auch die Gefahr der gefürchteten Dislokation des Zwischenteils bei der Flexion gebannt.

Die jeweilige Führungsbahn kann geradlinig verlaufen. Dies ermöglicht eine einfache Herstellung der Prothese.

Bei einem vorteilhaften Ausführungsbeispiel der Erfindung ist am zweiten Prothesenteil ein Drehzapfen ausgebildet, um welchen das Zwischenteil verschwenkbar gelagert ist. Dies ergibt eine besonders einfache Konstruktion.

Zweckmässigerweise besitzt das zweite Prothesenteil eine Gleitfläche, auf welcher das Zwischenteil gelagert ist. Diese Gleitfläche kann verhältnismässig gross gehalten werden, so dass die Flächenpressung klein bleibt und eine Abnützung praktisch vermieden wird.

Vorteilhaft sind Mittel vorgesehen, um ein Abheben des vom zweiten Prothesenteil zu verhindern. Weiter ist die Konstruktion vorzugsweise so gestaltet, dass die Beweglichkeit des Zwischenteils relativ zum Kupplungsorgan auf Bewegungen entlang der Führungsbahn eingeschränkt ist. So ist es möglich, dass Führungsbahn und Führungsteil als Nut und Steg, als Schwalbenschwanz, als Ueberdachung oder bogenförmige Umfassung ausgebildet sind. Durch diese Ausgestaltung wird verhindert, dass das Führungsteil die Führungsbahn verlässt, wenn aus irgendwelchen Gründen die Spannung der nach der Operation verbliebenen Gelenkbänder nachlässt.

Die Drehgelenkabschnitte können verschiedenartig ausgebildet sein, um eine Drehbewegung zu ermöglichen. Eine vorteilhafte Ausführungsform sieht vor, dass der Drehgelenkabschnitt des ersten Prothesenteils durch zwei Kondylen gebildet ist, und für jede dieser Kondylen ein Zwischenteil mit einer Lagerfläche für die Kondyle vorgesehen ist. Dies ergibt eine Prothese, die dem natürlichen Gelenkaufbau, wie er beispielsweise beim Kniegelenk zu finden ist, sehr nahe kommt.

Eine andere vorteilhafte Ausführungsform sieht vor, dass der Drehgelenkabschnitt des ersten Prothesenteils durch zwei Kondylen gebildet ist und dass ein für beide Kondylen gemeinsames Zwischenteil mit einer Lagerfläche für jeden der Kondylen vorgesehen ist. Auch diese Gestaltung ermöglicht einen Bewegungsablauf, der dem natürlichen physiologischen Bewegungsablauf sehr nahekommt. Es genügt dabei, für den gemeinsamen Zwischenteil eine einzige Führungsbahn vorzusehen. Es ist aber auch möglich, dass das gemeinsame Zwischenteil durch zwei in einem Abstand voneinander angeordnete Führungsbahnen geführt wird. Dies trägt dazu bei, eine Abnützung praktisch zu verhindern.

Möglich ist auch eine monokompartimentäre Ausführung der Gelenkprothese. So kann der Drehgelenkabschnitt des ersten Prothesenteils durch eine Kondyle gebildet sein und das Zwischenteil eine Lagerfläche für diese Kondyle aufweisen.

Wenn das Bedürnis besteht, auch bei gestörtem Bandapparat oder fehlenden Bändern Stabilität zu gewährleisten, kann das Kupplungsorgan ein Stabilisierungsglied aufweisen, welches mit einem Stabilisierungsorgan des ersten Prothesenteils zusammenarbeitet, um so auch bei fehlenden oder schwachen Bandstrukturen Stabilität zu gewährleisten. Eine solche Prothese ermöglicht einen Bewegungsablauf, der weitgehend dem normalen physiologischen Bewegungsablauf entspricht. So findet bei der Flexion eine Dreh- und eine Gleitbewegung statt. Gleichzeitig kann aber auch eine Rotationsbewegung des Unterschenkels stattfinden. Sowohl bei der Flexion als auch bei der Extension sind daher ähnliche Verhältnisse gegeben wie beim natürlichen Knie. Von oben kommende Kräfte wirken nicht voll auf das Interface, sondern werden durch den Zwischenteil in Translationsbewegung umgesetzt. Eine Varus- oder Valgusstellung wird durch das Zusammenwirken des Stabilisierungsglieds mit dem Stabilisierungsorgan vermieden. Auch ist die Gefahr der Dislokation des Zwischenteils bei der Flexion oder bei geschwächten oder fehlenden Bandstrukturen sicher gebannt.

Bei einer vorteilhaften Ausführungsform der Erfindung weist das Kupplungsorgan zur verschwenkbaren Lagerung einen Drehzapfen auf, welcher in einer Bohrung des zweiten Prothesenteils gelagert ist. Dies ergibt eine besonders einfache Konstruktion.

Der Drehgelenkabschnitt des ersten Prothesenteils und der Drehgelenkabschnitt des Zwischenteils sind konvex bzw. konkav und besitzen vorteilhaft die gleiche Wölbung. Dadurch wird in jeder Gelenkstellung die sogenannte Kongruenz der miteinander in Kontakt stehenden Gelenkflächen gewährleistet. Die Flächenpressung und damit der Verschleiss werden dadurch gering gehalten. Dies ermöglicht es, gegebenenfalls auf Teile aus Polyethylen zu verzichten, und verschleissresistente Hartstoff-Materialpaarungen, wie z.B. Metall/Metall, Keramik/Keramik, etc. vorzusehen. Ganz allgemein kann dadurch die Lebensdauer und die Biokompatibilität der Kniegelenkprothese wesentlich erhöht werden.

Eine vorteilhafte Ausführungsform sieht vor, dass der Drehgelenkabschnitt des ersten Prothesenteils zylindrisch gewölbt ist. Dies erlaubt eine einfache Herstellung des ersten Prothesenteils und des entsprechend geformten Zwischenteils. Wenn es erwünscht ist, näher beim natürlichen Vorbild des Kniegelenks zu bleiben, können am ersten Prothesenteil auch zwei kondylenartige, sphärisch gewölbte Flächen vorgesehen werden. Es ist aber auch möglich, den Drehgelenkabschnitt des ersten Prothesenteils sphärisch gewölbt über beide Kondylen gemeinsam auszubilden.

Das erste Prothesenteil kann einstückig sein. Zur Bildung von vorteilhaften Kniegelenkbausätzen ist es aber zweckmässig, eine zweiteilige Ausführung vorzusehen, d.h. das Stabilisierungsorgan als ein auf ein Basisteil aufsetzbares Bauteil zu gestalten, das dann nur bei Bedarf verwendet wird.

Für das erste Prothesenteil, das Zwischenteil und das zweite Prothesenteil können verschiedene Grössen vorgesehen werden, die aber mit dem gleichen Kupplungsorgan kombiniert werden können.

Ausführungsbeispiele der Erfindung werden nun unter Bezugnahme auf die Zeichnung beschrieben. Es zeigt:
- Figur 1: die Kraftumsetzung in Translationsbewegung bei einem Schlittengelenk,
- Figur 2: die Scherkraftbildung am Interface bei einem Scharniergelenk,
- Figur 3: die Verhältnisse bei einem "geöffneten" Scharniergelenk,
- Figur 4: die Verhältnisse bei einem kombinierten Schlitten-und Scharniergelenk,
- Figur 5: ein erstes Ausführungsbeispiel einer Prothese, z.B. Kniegelenkprothese, in auseinandergezogener Darstellung,
- Figur 6: eine Ansicht von oben auf das Zwischenteil,
- Figur 7: das Zwischenteil von Figur 6 von unten gesehen,
- Figur 8: das zweite Prothesenteil mit aufgesetztem Kupplungsorgan und Zwischenteil von oben gesehen,
- Figur 9a: einen Schnitt entlang der Linie IX-IX von Figur 8,
- Figur 9b: eine Explosionsdarstellung der Prothese von Figur 5 bis 9a,
- Figur 10: einen Schnitt durch ein zweites Ausführungsbeispiel einer Kniegelenkprothese,
- Figur 11: einen Schnitt durch ein drittes Ausführungsbeispiel einer Kniegelenkprothese,
- Figur 12a: einen Schnitt durch ein viertes Ausführungsbeispiel einer Kniegelenkprothese,
- Figur 12b: eine Explosionsdarstellung der Prothese von Figur 12a,
- Figur 13a: ein fünftes Ausführungsbeispiel einer Kniegelenkprothese im Schnitt, wobei das erste Prothesenteil durch eine gestrichelte Linie gezeigt ist,
- Figur 13b: eine Explosionsdarstellung der Prothese von 13a,
- Figur 14: eine Ansicht von oben auf die Kniegelenkprothese gemäss Figur 13a,
- Figur 15: einen Schnitt durch ein sechstes Ausführungsbeispiel einer Kniegelenkprothese,
- Figur 16: eine Ansicht von oben auf die Kniegelenkprothese von Figur 15,
- Figur 17a: einen Schnitt durch ein siebentes Ausführungsbeispiel einer Kniegelenkprothese,
- Figur 17b: eine Explosionsdarstellung der Prothese von Figur 17a,
- Figur 18: eine Ansicht von oben auf die Kniegelenkprothese von Figur 17a,
- Figur 19a: einen Schnitt durch ein achtes Ausführungsbeispiel einer Kniegelenkprothese und
- Figur 19b: eine Explosionsdarstellung der Prothese von Figur 19a,
- Figur 20: eine Ansicht von oben auf die Kniegelenkprothese von Figur 19a,
- Figur 21: eine Kniegelenkprothese mit Stabilisierungsmitteln in auseinander gezogenen Darstellung,
- Figur 22: die monozentrische Ausbildung der Gelenkfläche des Femurteils,
- Figur 23: die Kniegelenkprothese gemäss Figur 21 bei Extension,
- Figur 24: die Kniegelenkprothese von Figur 21 bei 90 Grad Flexion,
- Figuren 25 bis 27: Ausführungsbeispiele für die zylindrische bzw. sphärische Ausbildung der Drehgelenkabschnitte,
- Figur 28: ein letztes Ausführungsbeispiel einer Kniegelenkprothese.

Bei den bereits einleitend behandelten verschiedenen bekannten Gelenkformen der Figuren 1 bis 4 ist die Tibia mit der Bezugsziffer 1 bezeichnet. Auf der Tibia 1 ist das untere Gelenkteil 17 befestigt. Die Bezugsziffer 11 bezeichnet das obere Gelenkteil. In Figur 4 findet sich noch ein auf dem unteren Gelenkteil 17 verschiebbares Zwischenteil 13.

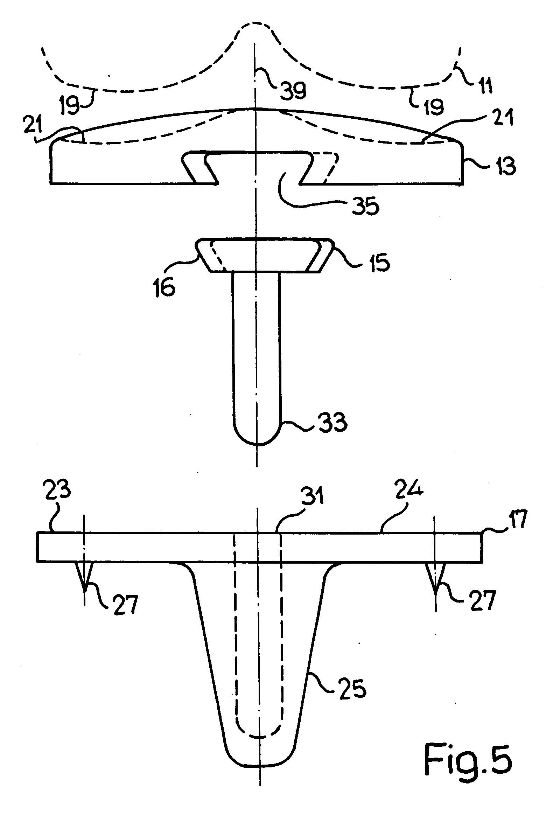

Die in den Figuren 5 bis 9 dargestellte Kniegelenkprothese besitzt ein erstes Prothesenteil 11, ein Zwischenteil 13, ein Kupplungsorgan 15 und ein zweites Prothesenteil 17.

Das erste Prothesenteil 11 kann in herkömmlicher Weise mindestens einen Verankerungsabschnitt und mindestens einen Drehgelenkabschnitt aufweisen. Für die vorliegende Erfindung ist natürlich von Bedeutung, dass der Drehgelenkabschnitt des ersten Prothesenteils mit jenem des ersten Zwischenteils 13 übereinstimmt. Wie in Figur 5 dargestellt, wird der Drehgelenkabschnitt des ersten Prothesenteils 11 durch zwei Kondylen 19 gebildet. Diesen Kondylen 19 entsprechen die Lagerflächen 21 des Zwischenteils 13.

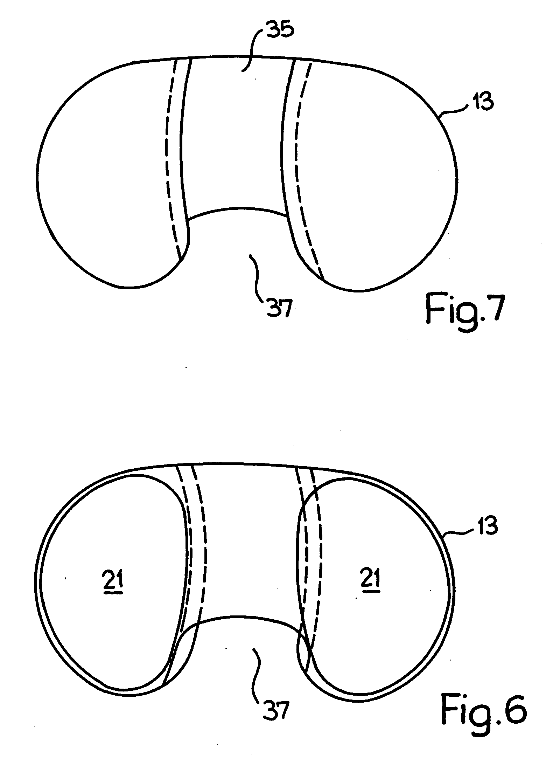

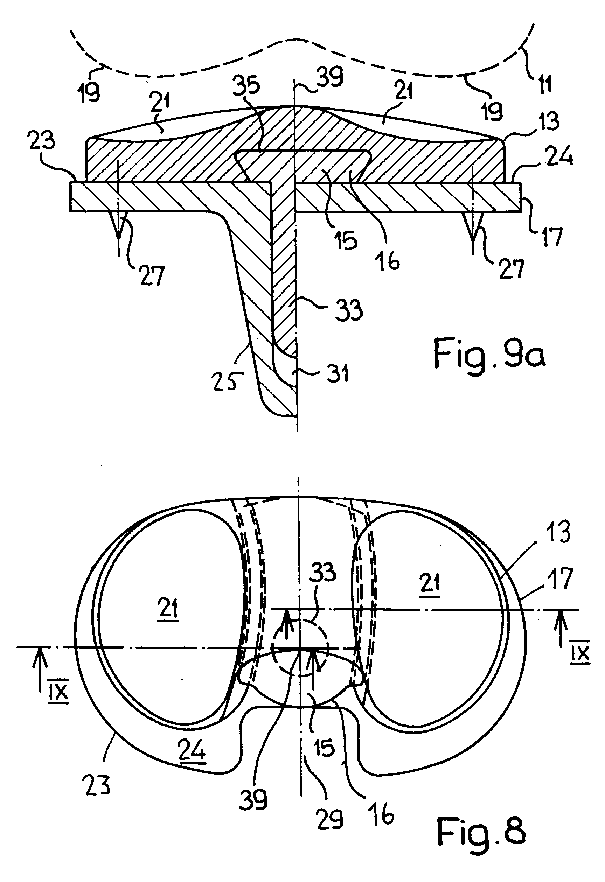

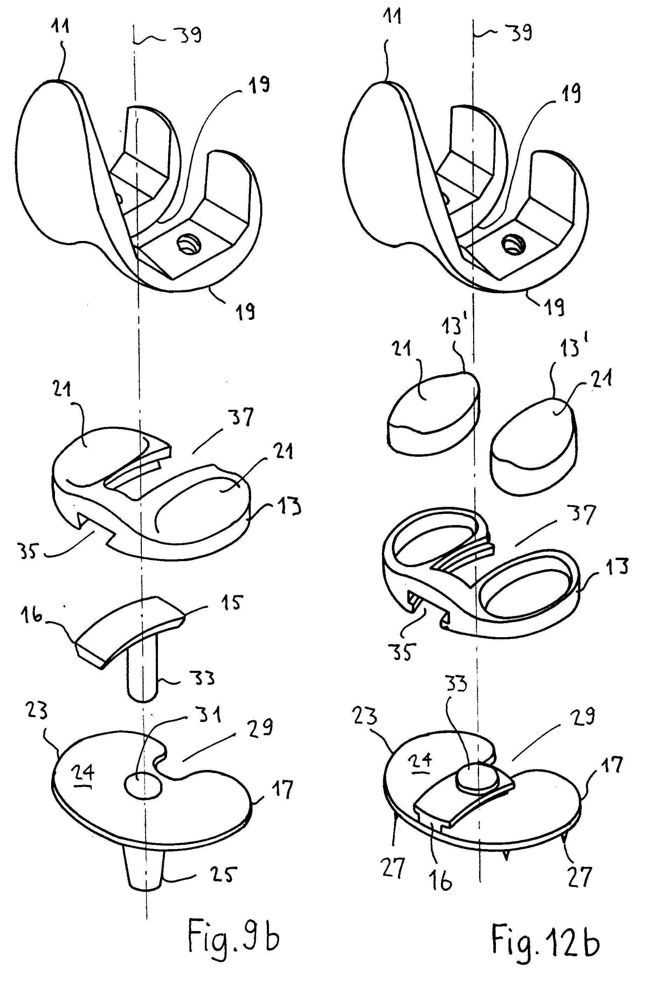

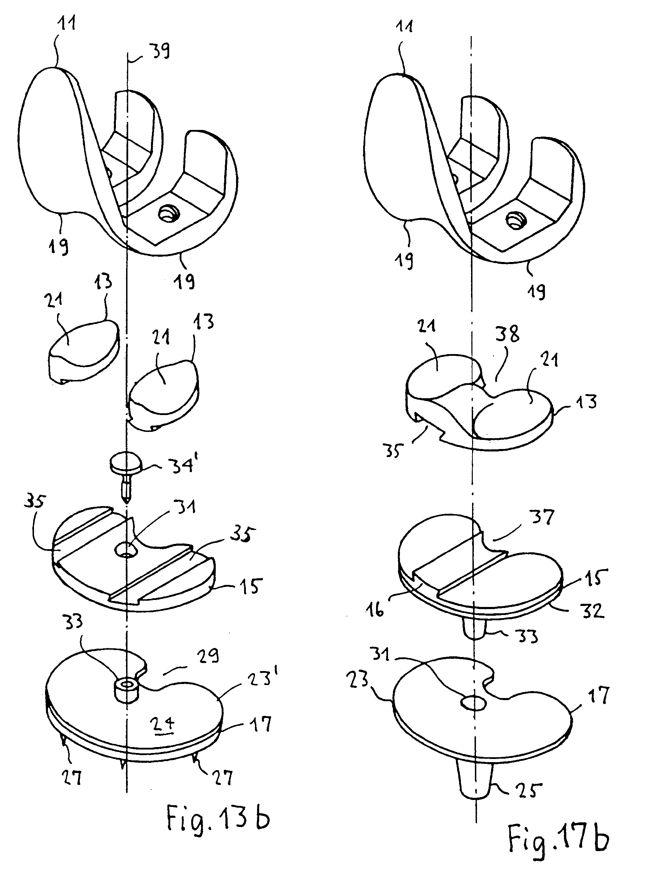

Das zweite Prothesenteil 17 besteht aus einer eine Gleitfläche 24 aufweisenden Platte 23, von welcher sich konische Verankerungsabschnitte 25, 27 nach unten erstrecken. Wie Figur 8 zeigt, besitzt die Platte 23 eine den Umrissen der natürlichen Tibiakondylen angepasste ovale Formgebung mit einer posterioren Ausnehmung 29 für das hintere Kreuzband. Der Verankerungsabschnitt 25 ist relativ gross und weist eine Bohrung 31 zur Aufnahme des Drehzapfens 33 des Kupplungsorgans 15 auf. Im Zwischenteil 13 befindet sich eine Führungsbahn 35 in Form einer Schwalbenschwanz-Nut. Sie dient der Aufnahme des Führungsteils 16 des Kupplungsorgans 15. Führungsbahn 35 und Führungsteil 16 bilden eine Einrichtung, um dem Zwischenteil 13 zusätzlich zur Schwenkbewegung auch eine Translationsbewegung zu ermöglichen. Für das Zusammenwirken von 35 und 16 ist von untergeordneter Bedeutung, welches als Nut und welches als Schiene ausgebildet ist. Es ist daher immer der zum Kupplungsorgan gehörende Führungsteil mit der Bezugsziffer 16 bezeichnet (Figuren 15, 16 und 17). Statt Schwalbenschwanzform könnte die Einrichtung 35, 16 aber auch einen anderen geeigneten Querschnitt aufweisen. Die Führungsbahn 35 ist praktisch mittig im Zwischenteil 13 angeordnet und verläuft ungefähr sagittal in einem nach medial gekrümmten Bogen, wie dies beispielsweise aus der Ansicht von unten gemäss Figur 7 ersichtlich ist.

Wie Figuren 8 und 9b zeigen, hat das Zwischenteil 13 eine ähnliche Formgebung wie die Platte 23, ist aber in sagittaler Richtung wesentlich schmäler als die Platte 23. Das Zwischenteil 13 weist ebenfalls eine Ausnehmung 37 für das hintere Kreuzband auf.

Bei einer Betrachtung der Figuren 8, 9a und 9b kann man erkennen, dass das Zwischenteil 13 sowohl eine Schwenkbewegung um die Achse 39 als auch eine Translationsbewegung von anterior nach posterior und umgekehrt durchführen kann. Diese Bewegungen werden nach dem Einbau der Prothese durch die Bänder des Kniegelenks geführt und begrenzt. Der Bewegungsablauf bei Flexion und Extension entspricht weitgehend dem physiologischen Bewegungsablauf des Kniegelenks.

Das erste und das zweite Prothesenteil 11, 17 und das Kupplungsorgan 15 mit dem Drehzapfen 33 bestehen vorteilhaft aus einer Metallegierung, wie sie üblicherweise für Gelenkprothesen Anwendung findet. Das Zwischenteil 13 besteht zweckmässigerweise aus einem Kunststoff, wie er ebenfalls üblicherweise für Gelenkprothesen verwendet wird.

Beim gezeigten Ausführungsbeispiel kommen zwei Kondylen/Lagerflächen-Paare 19, 21 zur Anwendung. Es ist aber auch möglich, nur ein Kondylen/Lagerflächen-Paar zu verwenden. Denkbar wäre auch eine andere gelenkige Verbindung, z.B. ein durch einen Achsbolzen gekoppeltes Scharniergelenk, in Kombination mit der eine Translation und Rotation ermöglichenden Ausgestaltung des Zwischenteils 13 und des Kupplungsorgans 15.

Beim gezeigten Ausführungsbeispiel sind die Kondylen 19 des ersten Prothesenteils 11 konvex und die Lagerflächen 21 des Zwischenteils 13 entsprechend konkav. Es wäre aber auch möglich, z.B. dort, wo es die Natur nahelegt, z.B. beim Patella-Femur-Gelenk im ersten Prothesenteil statt Kondylen, konkave Lagerflächen und im Zwischenteil statt konkave Lagerflächen konvexe Kondylen vorzusehen.

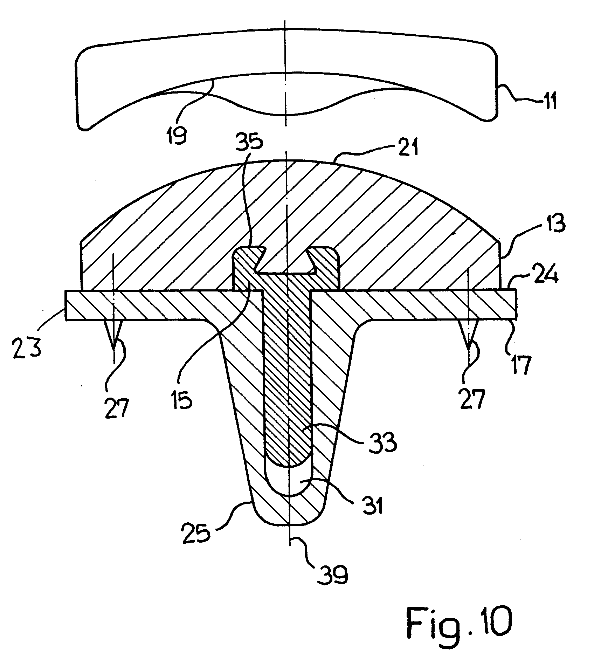

Beim Ausführungsbeispiel von Figur 10 ist nur eine Kondyle 19 und eine Lagerfläche 21 vorgesehen. Im übrigen ist die Anordnung ähnlich wie unter Bezugnahme auf die Figuren 5 bis 9a und 9b beschrieben. Die Lagerfläche 19 des ersten Prothesenteils ist jedoch konkav und die entsprechende Lagerfläche 21 des Zwischenteils 13 ist konvex. Eine solche Prothese eignet sich für das Patella-Femur-Gelenk.

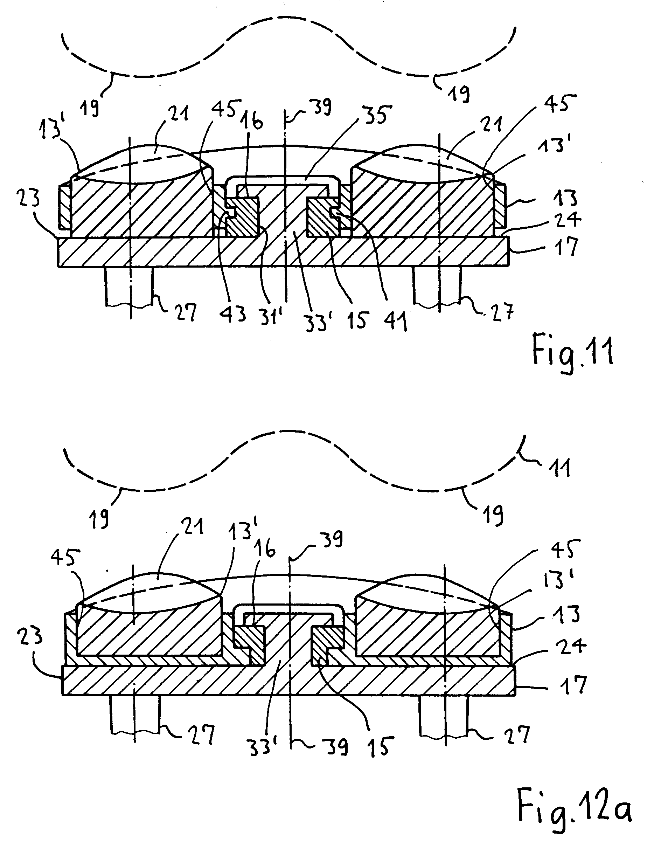

Beim Ausführungsbeispiel von Fig. 11 ist das zweite Prothesenteil 17 mit einem Drehzapfen 33' versehen, um welchen sich das Kupplungsorgan 15 drehen kann. Dieses weist zur Aufnahme des Drehzapfens eine Bohrung 31' auf. Der Führungsteil 16 des Kupplungsorgans 15 besitzt auf beiden Seiten eine Nut 41 für eine Rippe 43 des Zwischenteils 13. Nut und Rippe können aber auch vertauscht angeordnet sein. Auf diese Weise wird das Zwischenteil 13 in einem Abstand von der Platte 23 gehalten. Die Lagerflächen 21 sind als auswechselbare Lagerelemente 13' ausgebildet, welche in Aussparungen 45 des ersten Zwischenteils 13 eingelassen sind und auf der Platte 23 aufliegen. Bei einer Dreh- und/oder Translationsbewegung des Zwischenteils 13 gleiten daher die Lagerelemente 13' auf der Platte 23. Die Lagerelemente 13' bestehen vorteilhaft aus einem für Implantate üblichen Kunststoff. Das Zwischenteil 13 kann aus Metall bestehen, während das Kupplungsorgan 15 zweckmässigerweise aus Kunststoff besteht. Es ist also möglich, immer eine Paarung von Kunststoff und Metall bei den verschiedenen Reibflächen vorzusehen. Weitere Paarungsmöglichkeiten werden später noch näher beschrieben.

Die Ausführungsform der Prothese gemäss den Figuren 12a und 12b unterscheidet sich von der Ausführungsform von Figur 11 im wesentlichen dadurch, dass die Aussparungen 45 nicht durchgehend sind und das Zwischenteil 13 auf der Gleitfläche 24 der Platte 23 aufliegt. Es kann daher in bezug auf die übrigen Details und die Funktionsweise auf die vorangehende Beschreibung verwiesen werden.

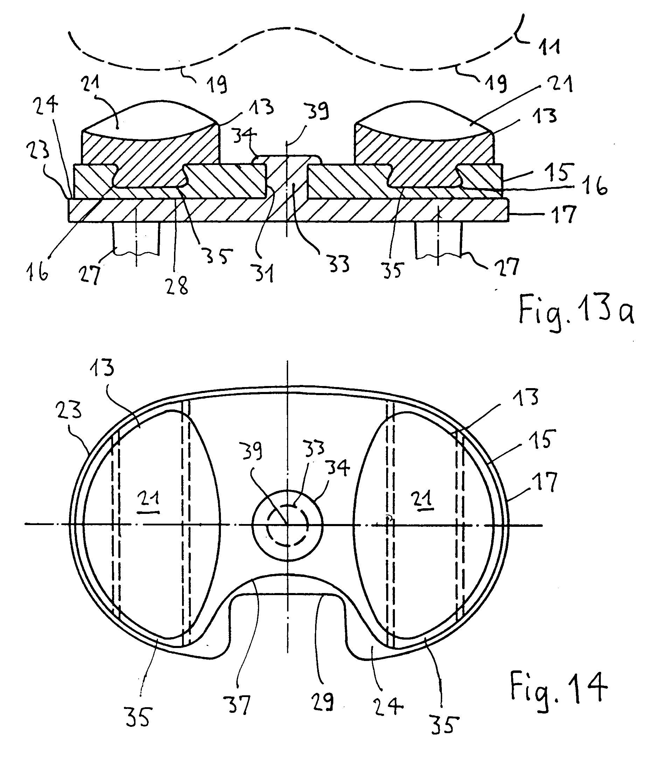

Die in den Figuren 13a, 13b und 14 dargestellte bikompartimentäre Kniegelenkprothese besitzt ein erstes Prothesenteil 11, ein Kupplungsorgan 15, zwei Zwischenteile 13 und ein zweites Prothesenteil 17.

Das erste Prothesenteil 11 kann in herkömmlicher Weise mindestens einen Verankerungsabschnitt und mindestens einen Drehgelenkabschnitt aufweisen. Für die vorliegende Erfindung ist natürlich von Bedeutung, dass der Drehgelenkabschnitt 19 des ersten Prothesenteils 11 mit jenem des Zwischenteils 13 übereinstimmt. Wie in Figuren 13a, 13b dargestellt, wird der Drehgelenkabschnitt des ersten Prothesenteils 11 durch zwei Kondylen 19 gebildet. Diesen Kondylen 19 entsprechen die Lagerflächen 21 der Zwischen- oder Meniskusteile 13.

Das zweite Prothesenteil 17 besteht aus einer eine Gleitfläche 24 aufweisenden Platte 23, von welcher sich konische Verankerungsabschnitte 27 nach unten erstrecken. In Fig. 13b wird gezeigt, dass die Oberseite der Platte 23 eine Schicht 23' aus Kunststoff aufweisen kann. Wie Figur 14 zeigt, besitzt die Platte 23 eine den Umrissen der natürlichen Tibiakondylen angepasste ovale Formgebung mit einer posterioren Ausnehmung 29 für das hintere Kreuzband. Das zweite Prothesenteil 17 weist einen Drehzapfen 33 auf, welcher in eine Bohrung 31 des Kupplungsorgans 15 passt. Eine Ueberdachung 34 verhindert ein Abheben des Zwischenteils 13 von der Gleitfläche 24. Es kann auch z.B. eine Schraube 34' (Fig. 13b) oder dergleichen vorgesehen sein. Im Kupplungsorgan 15 befinden sich zwei Führungen 16 in Form von Schwalbenschwanz-Nuten. Sie könnten aber auch einen anderen geeigneten Querschnitt aufweisen. Die Führungen 16 sind praktisch parallel zueinander im Zwischenteil 15 angeordnet und verlaufen ungefähr sagittal.

Wie Figur 14 zeigt, hat das Kupplungsorgan 15 eine ähnliche Formgebung wie die Platte 23, ist aber in sagittaler Richtung schmäler als die Platte 23. Das Zwischenteil 13 weist ebenfalls eine Ausnehmung 37 für das hintere Kreuzband auf.

Bei einer Betrachtung der Figuren 13a und 14 kann man erkennen, dass das Kupplungsorgan 15 eine Schwenkbewegung um die Achse 39 und die Zwischenteile 13 eine Translationsbewegung von anterior nach posterior und umgekehrt durchführen können. Diese Bewegungen werden nach dem Einbau der Prothese durch die Bänder des Kniegelenks geführt und begrenzt. Der Bewegungsablauf bei Flexion und Extension entspricht weitgehend dem physiologischen Bewegungsablauf des Kniegelenks.

Das erste und das zweite Prothesenteil 11, 17 sowie das Kupplungsorgan 15 bestehen vorteilhaft aus einer Metallegierung, wie sie üblicherweise für Gelenkprothesen Anwendung findet. Die Zwischenteile 13 bestehen zweckmässigerweise aus einem Kunststoff, wie er ebenfalls üblicherweise für Gelenkprothesen verwendet wird. Es ist jedoch auch möglich, wegen der hohen Kongruenz der Lagerflächen 21 und der Kondylen 19, die Zwischenteile 13 ebenfalls aus Metall zu fertigen. Andererseits kann jedoch auch, wo dies opportun erscheint, eine der beiden zueinander gewandten Gleitflächen 24, 28 des zweiten Prothesenteils 17, bzw. des Kupplungsorgans 15 mit Kunststoff belegt werden. Es ist also möglich, immer eine Paarung von Kunststoff und Metall bei den verschiedenen Gleitflächen vorzusehen oder sämtliche Gleitflächen in Metall- oder anderer zweckmässiger Hartstoffpaarung auszuführen.

Beim gezeigten Ausführungsbeispiel kommen zwei Kondylen/Lagerflächen-Paare 19, 21 zur Anwendung. Es ist aber auch möglich, nur ein Kondylen/Lagerflächen-Paar zu verwenden.

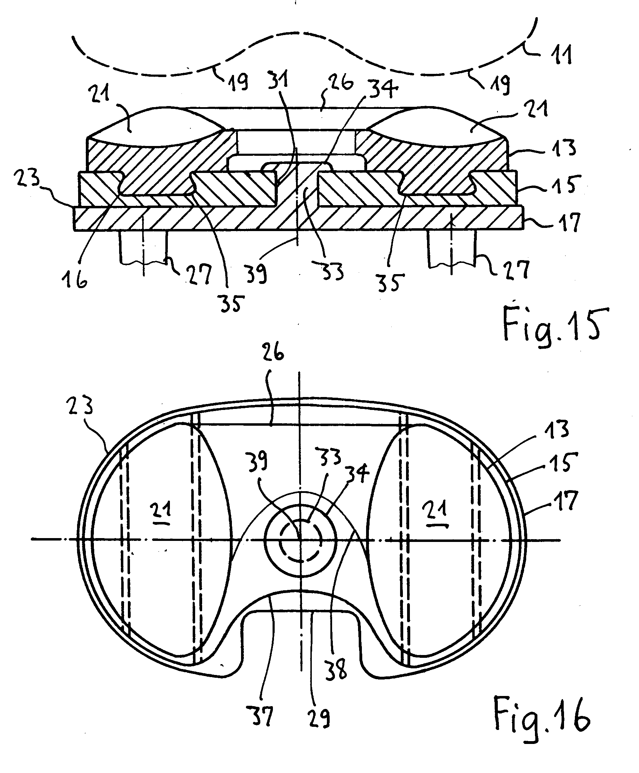

Die Ausführungsform gemäss den Figuren 15 und 16 unterscheidet sich von jener von Figur 13a, 13b und 14 dadurch, dass für beide Kondylen 19 ein gemeinsames Zwischenteil 13 vorgesehen ist, welches eine Ausnehmung 38 für das hintere Kreuzband aufweist. Man kann sich also die beiden Zwischenteile 13 der Figuren 13a, 13b und 14 durch eine Brücke 26 (Figur 15) verbunden vorstellen. Zweckmässigerweise ist aber das gemeinsame Zwischenteil 13 von Figur 15 einstückig. Er weist zwei Lagerflächen 21 auf. Im übrigen ist die Prothese gleich ausgebildet wie in den Figuren 13a, 13b und 14, so dass für Einzelheiten auf die vorherige Beschreibung verwiesen werden kann.

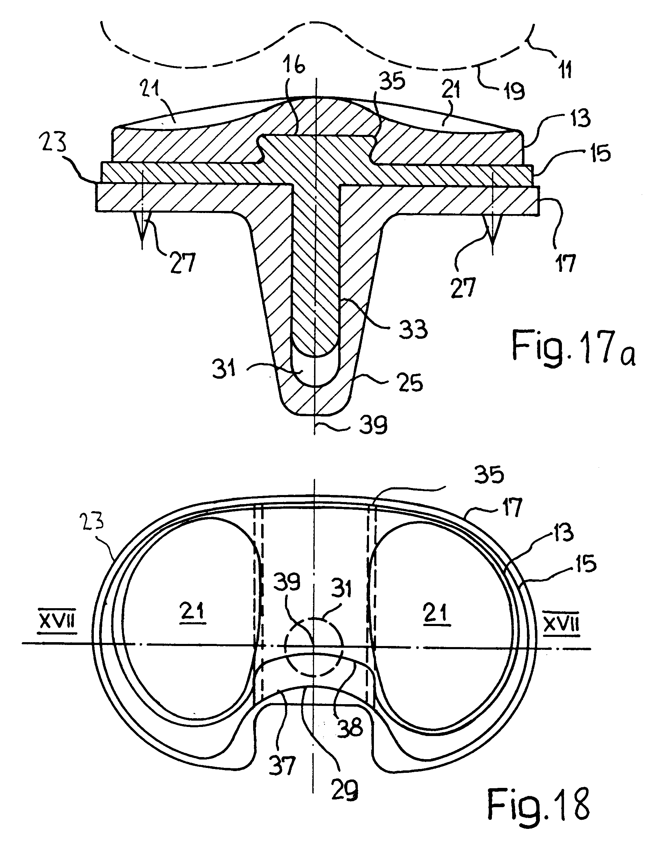

Auch bei der Ausführungsform gemäss den Figuren 17a, 17b und 18 ist für beide Kondylen 19 ein gemeinsames Meniskusteil 13 vorgesehen. Ein Unterschied zu den vorher beschriebenen Ausführungsbeispielen besteht aber darin, dass nicht das zweite Prothesenteil 17, sondern das Kupplungsorgan 15 einen Drehzapfen 33 aufweist, welcher in einer Bohrung 31 des relativ grossen Verankerungsabschnitts 25 gelagert ist. Die konischen Verankerungsabschnitte 27 sind relativ klein ausgebildet. Ein weiterer Unterschied besteht darin, dass das Führungsteil 16 des Kupplungsorgans 15 als Steg ausgebildet ist und die Führungsbahn 35 des Zwischenteils 13 als eine den Steg umfassende Nut. Auch das Zwischenteil 13 weist dorsal eine Ausnehmung 38 für das hintere Kreuzband auf. In Fig. 17b wird gezeigt, dass die Unterseite des Kupplungsorgans 15 eine Schicht 32 aus Kunststoff aufweisen kann. Im übrigen wird wiederum auf die Beschreibung in bezug auf die Figuren 13a, 13b und 14 verwiesen.

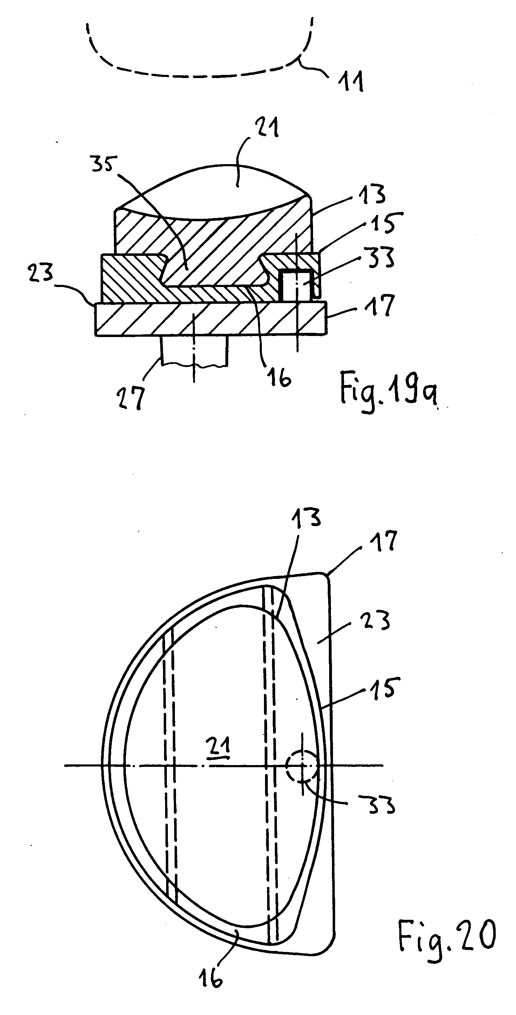

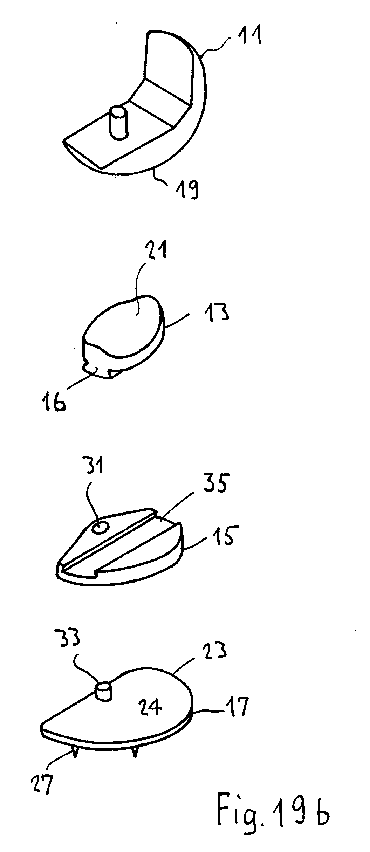

In den Figuren 19a, 19b und 20 ist eine monokompartimentäre Gelenkprothese dargestellt, welche grundsätzlich den gleichen Aufbau aufweist, wie die bikompartimentäre Gelenkprothese gemäss den Figuren 5 und 6. Es kann daher auch auf die vorangehende Beschreibung verwiesen werden. Wenn in Fig. 20 keine Mittel dargestellt sind welche analog zur Ueberdachung 34 (Fig. 13a) ein Abheben des Kupplungsorgans verhindern, so ist doch dem Fachmann klar, dass durch geeignete Massnahmen, z.B. durch Anbringen eines Seegerrings, ein Abheben verhindert werden kann.

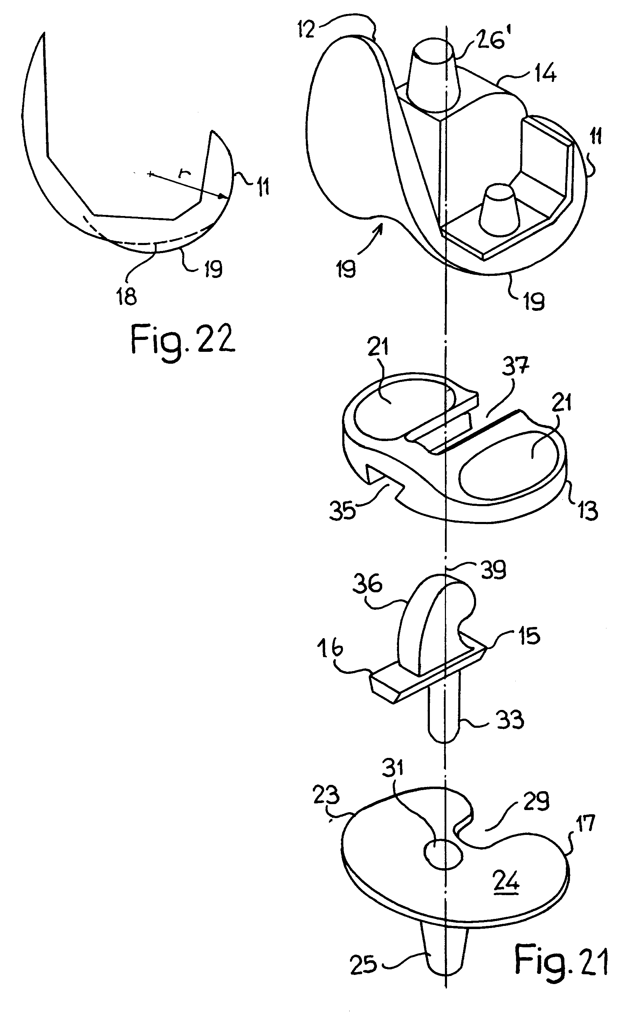

Die Kniegelenkprothese gemäss Fig. 21 besitzt ein erstes Prothesen- oder Femurteil 11, ein erstes Zwischen- oder Meniskusteil 13, ein zweites Zwischenteil oder Kupplungsorgan 15 und ein zweites Prothesen- oder Tibiateil 17. Das erste Prothesenteil 11 ist zweistückig. Es besteht aus dem Basisteil 12 und dem darauf aufgesetzten Stabilisierungsorgan 14, welche fest miteinander verbunden sind. Die so gebildete Einheit besitzt einen Verankerungsabschnitt 26' zur Verankerung im Femur und einen Drehgelenkabschnitt, welcher durch zwei kondylenartige sphärisch gewölbte Flächen 19 gebildet wird. Wie insbesondere Fig. 22 zeigt, besitzen die konvexen, z.B. sphärischen, Flächen 19 den Radius R. Dies weicht von der mehr elliptischen Form des natürlichen Knies ab. Die Form der natürlichen Kondylen ist gestrichelt angedeutet.

Den sphärischen Flächen 19 entsprechen die konkaven Lagerflächen 21, welche ebenfalls den Radius r besitzen, um so die sogenannte Kongruenz zu gewährleisten welche verschleissminimierend wirkt. Das zweite Prothesenteil 17 besteht aus einer eine Gleitfläche 24 aufweisenden Platte 23, von welcher sich der konische Verankerungsabschnitt 25 nach unten erstreckt. Die Platte 23 besitzt eine den Umrissen der natürlichen Tibiakondylen angepasste ovale Formgebung mit einer posterioren Ausnehmung 29 für das hintere Kreuzband. Der Verankerungsabschnitt 25 ist relativ gross und weist eine Bohrung 31 zur Aufnahme des Drehzapfens 33 des Führungsorgans 15 auf. Im Zwischenteil 13 befindet sich eine Führungsbahn 35 in Form einer Schwalbenschwanznut. Sie könnte aber auch einen anderen geeigneten Querschnitt aufweisen. Die Führungsbahn 35 ist praktisch mittig im Meniskusteil 13 angeordnet und verläuft ungefähr in Richtung anterior-posterior. Statt gerade, könnte sie auch in einem vorzugsweise nach medial gekrümmten Bogen verlaufen. Die gerade Ausführung hat jedoch den Vorteil, dass das Zwischenteil 13 sowohl für das linke als auch für das rechte Kniegelenk brauchbar ist und die Gefahr einer Verwechslung ausgeschlossen wird.

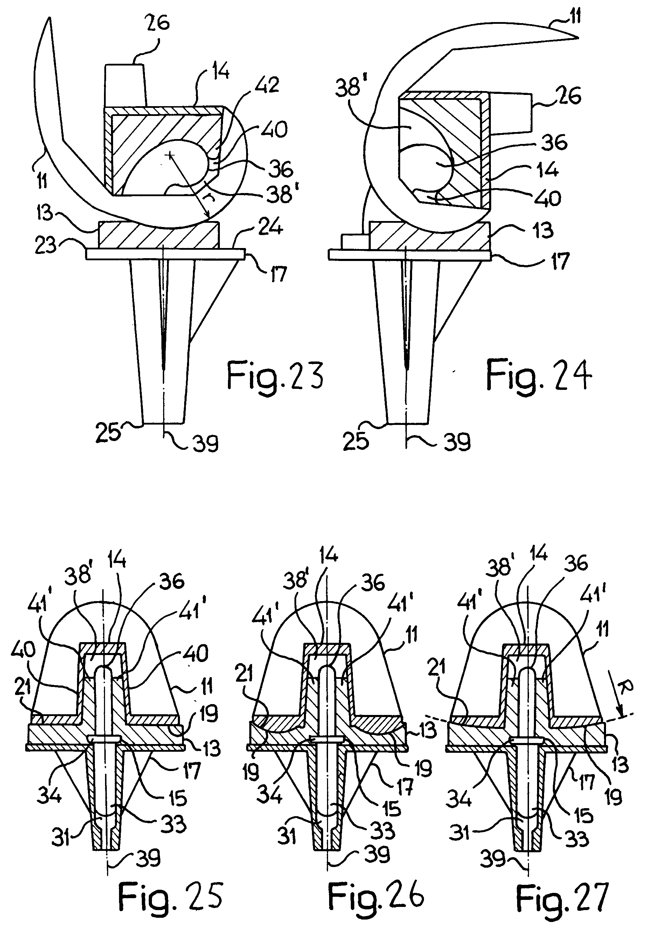

Das Zwischenteil 13 hat eine ähnliche Formgebung wie die Platte 23, ist aber in sagittaler Richtung schmäler als die Platte 23. Das Zwischenteil 13 weist ebenfalls eine Ausnehmung 37 für das hintere Kreuzband auf. Das Kupplungsorgan 15 besitzt ein Führungsteil 16, welches in die Schwalbenschwanznut 35 des Zwischenteils 13 passt. Vom Führungsteil 16 erstreckt sich der Drehzapfen 33 nach unten und das Stabilisierungsglied 36 nach oben. Wie aus den Figuren 23 und 24 ersichtlich ist, arbeitet das Stabilisierungsglied 36 mit dem Stabilisierungsorgan 14 des ersten Prothesenteils 11 zusammen, um auch bei fehlenden oder schwachen Bandstrukturen Stabilität zu gewährleisten. Wie aus Fig. 23 ersichtlich ist, greift das Stabilisierungsglied 36 in das Innere 38' des Kastens 14 ein, wo es von den seitlichen Kastenwänden 40 geführt wird. Dadurch wird eine Varus- oder Vagusstellung verhindert. Durch die Nase 42 wird auch Stabilität nach posterior gewährleistet, d.h. die Bewegung des Tibiateils 17 wird nach posterior hin begrenzt.

Das erste Prothesenteil 11, das zweite Prothesenteil 17 und das Kupplungsorgan 15 bestehen vorteilhaft aus einer Metallegierung, wie sie üblicherweise für Gelenkprothesen Anwendung findet. Das Zwischenteil 13 kann aus einem Kunststoff bestehen, wie er ebenfalls üblicherweise für Gelenkprothesen verwendet wird. Es kann aber auch aus einer Metallegierung bestehen, weil dank der beschriebenen kongruenten Ausbildung der Drehgelenkabschnitte 19, 21 die Flächenpressung und somit auch der Reibungsverschleiss klein gehalten werden kann. Möglich ist aber auch die Verwendung von Keramik für alle Teile, wobei aber eine Metallegierung vorzuziehen ist, weil sie mehr duktil ist.

Bei einer Betrachtung der Figuren 21, 23 und 24 kann man erkennen, dass das Zwischenteil 13 bei der Flexion sowohl eine Schwenkbewegung um die Achse 39 als auch eine Translationsbewegung von anterior nach posterior und umgekehrt durchführen kann. Der Bewegungsablauf entspricht dabei weitgehend dem physiologischen Bewegungsablauf eines intakten menschlichen Kniegelenks.

Weitere Ausführungsformen des erfindungsgemässen Kniegelenks sind in den Figuren 25 bis 27 dargestellt. Bei diesen Ausführungsformen erstrecken sich vom Zwischenteil 13 seitliche Wände 41' in das Innere 38' des kastenförmigen Stabilisierungsorgans 14 und verhindern so einen direkten Kontakt des Stabilisierungsglieds 36 mit den Seitenwänden 40. Bei Fig. 25 ist der Drehgelenkabschnitt 19 zylindrisch gewölbt mit einem Radius r (Fig. 23). In Fig. 26 sind wie in Fig. 21 zwei kondylenartige sphärisch gewölbte Flächen 19 vorgesehen. Demgegenüber besitzen in Fig. 7 die sphärisch gewölbten Flächen 19 einen gemeinsamen Radius R über beide Kondylen.

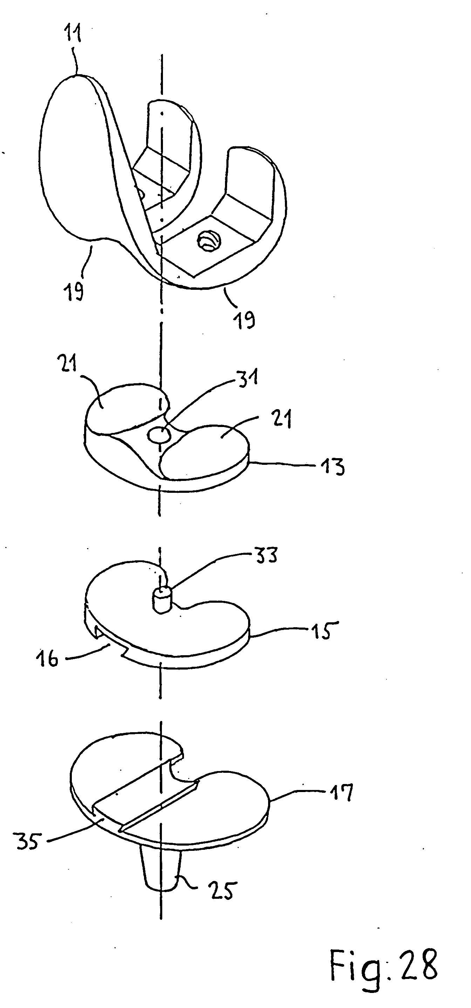

Beim Ausführungsbeispiel nach Fig. 28 ist der Drehzapfen 33 am Kupplungsorgan 15 angeordnet und das Zwischenteil 13 ist auf dem Drehzapfen 33 verschwenkbar gelagert.

## Patentansprüche

1. Gelenkprothese, insbesondere Kniegelenkprothese, mit
mindestens einem ersten Prothesenteil (11), das einen Verankerungsabschnitt und mindestens einen Drehgelenkabschnitt (19) aufweist,
einem zweiten Prothesenteil (17), das mindestens einen Verankerungsabschnitt (25, 27) und eine Gleitfläche (24) aufweist,
einem Zwischenteil (13), welches um einen Drehzapfen (33) verschwenkbar ist und mindestens einen Drehgelenkabschnitt (21) zur Zusammenarbeit mit dem entsprechenden Drehgelenkabschnitt (19) des ersten Prothesenteils (11) aufweist und in bezug auf den zweiten Prothesenteil (17) sowohl eine Translationsbewegung als auch eine Schwenkbewegung ausführen kann,
einem Kupplungsorgan (15), welches zwischen dem zweiten Prothesenteil (17) und dem Zwischenteil (13) angeordnet ist,
wobei das Zwischenteil (13) und das Kupplungsorgan (15) mittels eines Führungsteils (16) und einer nicht als Langloch ausgebildeten Führungsbahn (35), welche das Führungsteil aufnimmt und in welcher dieses medial und lateral geführt gleiten kann, zusammenwirken und entweder das Führungsteil (16) oder die Führungsbahn (35) am Kupplungsorgan (15) angeordnet ist und das entsprechende Gegenstück, nämlich Führungsbahn bzw. Führungsteil, am Zwischenteil (13) ausgebildet ist.

2. Gelenkprothese nach Anspruch 1, dadurch gekennzeichnet, dass der Drehzapfen (33) am Kupplungsorgan (15) angeordnet und im zweiten Prothesenteil (17) verschwenkbar gelagert ist.

3. Gelenkprothese nach Anspruch 2, dadurch gekennzeichnet, dass der Drehzapfen (33) in einer Bohrung (31) gelagert ist, die sich in einen Verankerungsabschnitt (25) erstreckt.

4. Gelenkprothese nach Anspruch 1, dadurch gekennzeichnet, dass der Drehzapfen (33') am zweiten Prothesenteil (17) angeordnet ist und dass das Kupplungsorgan (15) auf dem Drehzapfen (33') verschwenkbar gelagert ist.

5. Gelenkprothese nach Anspruch 1, dadurch gekennzeichnet, dass der Drehzapfen (33) am Kupplungsorgan (15) angeordnet ist, und dass das Zwischenteil (13) auf dem Drehzapren (33) verschwenkbar gelagert ist.

6. Gelenkprothese nach Anspruch 1, dadurch gekennzeichnet, dass der Drehzapfen (33) am zweiten Prothesenteil (17) angeordnet ist, und dass das Zwischenteil (13) auf dem Drehzapfen verschwenkbar gelagert ist.

7. Gelenkprothese nach einem der Ansprüche 1 bis 6, dadurch gekennzeichnet, dass die Führungsbahn (35) in sagittaler Richtung angeordnet ist.

8. Gelenkprothese nach Anspruch 7, dadurch gekennzeichnet, dass die jeweilige Führungsbahn (35) geradlinig verläuft.

9. Gelenkprothese nach Anspruch 7, dadurch gekennzeichnet, dass die Führungsbahn (35) in einem Bogen mit medialem Krümmungsradius verläuft.

10. Gelenkprothese nach einem der Ansprüche 1 bis 9, dadurch gekennzeichnet, dass das zweite Prothesenteil (17) eine Gleitfläche (24) aufweist, auf welcher das Zwischenteil (13) gelagert ist.

11. Gelenkprothese nach einem der Ansprüche 1 bis 10, dadurch gekennzeichnet, dass Mittel (34, 34') vorgesehen sind, um ein Abheben des Zwischenteils (13) vom zweiten Prothesenteil (17) zu verhindern.

12. Gelenkprothese nach einem der Ansprüche 1 bis 11, dadurch gekennzeichnet, dass die Beweglichkeit des Zwischenteils (13) relativ zum Kupplungsorgan (15) auf Bewegungen entlang der Führungsbahn (35) eingeschränkt ist.

13. Gelenkprothese nach einem der Ansprüche 1 bis 12, dadurch gekennzeichnet, dass die Führungsbahn (35) und Führungsteil (16) als Nut und Steg, als Schwalbenschwanz, als Ueberdachung oder bogenförmige Umfassung ausgebildet sind.

14. Gelenkprothese nach einem der Ansprüche 1 bis 13, dadurch gekennzeichnet, dass die Führungsbahn (35) praktisch mittig im Zwischenteil (13) angeordnet ist.

15. Gelenkprothese nach einem der Ansprüche 1 bis 14, dadurch gekennzeichnet, dass der Drehgelenkabschnitt des ersten Prothesenteils durch zwei Kondylen (19) gebildet ist und dass der Drehgelenkabschnitt des Zwischenteils durch zwei entsprechende Lagerflächen (21) zur Zusammenarbeit mit den Kondylen (19) des ersten Prothesenteils (11) gebildet ist.

16. Gelenkprothese nach einem der Ansprüche 1 bis 15, dadurch gekennzeichnet, dass der Drehgelenkabschnitt des ersten Prothesenteils (11) durch zwei Kondylen (19) gebildet ist und dass ein für beide Kondylen (19) gemeinsames Zwischenteil (13) mit einer Lagerfläche (21) für jede der Kondylen (19) vorgesehen ist.

17. Gelenkprothese nach Anspruch 16, dadurch gekennzeichnet, dass das gemeinsame Zwischenteil (13) durch zwei in einem Abstand voneinander angeordnete Führungsbahnen (16) geführt wird.

18. Gelenkprothese nach einem der Ansprüche 1 bis 15, dadurch gekennzeichnet, dass der Drehgelenkabschnitt des ersten Prothesenteils (11) durch eine Kondyle (19) gebildet ist und dass der Drehgelenkabschnitt des Zwischenteils (13) durch eine Lagerfläche (21) zur Zusammenarbeit mit der Kondyle (19) des ersten Prothesenteils (11) gebildet ist.

19. Gelenkprothese nach einem der Ansprüche 1 bis 18, dadurch gekennzeichnet, dass die jeweilige Kondyle (19) des ersten Prothesenteils (11) konvex und die zugehörige Lagerfläche (21) des Zwischenteils (13) entsprechend konkav ist.

20. Gelenkprothese nach einem der Ansprüche 1 bis 18, dadurch gekennzeichnet, dass die jeweilige Kondyle (19) des ersten Prothesenteils (11) konkav und die zugehörige Lagerfläche (21) des Zwischenteils (13) entsprechend konvex ist.

21. Gelenkprothese nach einem der Ansprüche 1 bis 20, dadurch gekennzeichnet, dass die jeweilige Lagerfläche (21) des Zwischenteils (13) durch ein in einer Aussparung (45) desselben auswechselbares Lagerelement (13') gebildet ist.

22. Gelenkprothese nach Anspruch 21, dadurch gekennzeichnet, dass die Aussparung (45) durchgehend ist.

23. Gelenkprothese nach Anspruch 21, dadurch gekennzeichnet, dass die Aussparung (45) nicht durchgehend ist.

24. Gelenkprothese nach einem der Ansprüche 1 bis 23, dadurch gekennzeichnet, dass das Zwischenteil (13) auf der Gleitfläche (24) verschiebbar gelagert ist.

25. Gelenkprothese nach einem der Ansprüche 1 bis 23, dadurch gekennzeichnet, dass das Zwischenteil (13) z.B. durch das Kupplungsorgan (15) in einem Abstand von der Gleitfläche (24) gehalten wird.

26. Gelenkprothese nach einem der Ansprüche 1 bis 25, dadurch gekennzeichnet, dass das zweite Prothesenteil (17) und das Zwischenteil (13) eine Ausnehmung (29, 37) für das vordere und/oder hintere Kreuzband aufweist.

27. Gelenkprothese nach Anspruch 1 oder 2, dadurch gekennzeichnet, dass das Kupplungsorgan (15) ein Stabilisierungsglied (36) besitzt, welches mit einem Stabilisierungsorgan (14) des ersten Prothesenteils (11) zusammenarbeitet, um auch bei fehlenden oder schwachen Bandstrukturen Stabilität zu gewährleisten.

28. Gelenkprothese nach Anspruch 27, dadurch gekennzeichnet, dass der Drehgelenkabschnitt (19) des ersten Prothesenteils (11) zylindrisch ist.

29. Gelenkprothese nach Anspruch 27, dadurch gekennzeichnet, dass der Drehgelenkabschnitt (19) des ersten Prothesenteils (11) sphärisch über beide Kondylen gewölbt ist.

30. Gelenkprothese nach Anspruch 27, dadurch gekennzeichnet, dass der Drehgelenkabschnitt (19) des ersten Prothesenteils (11) zwei kondylenartig sphärisch gewölbte Flächen (19) aufweist.

31. Gelenkprothese nach einem der Ansprüche 27 bis 30, dadurch gekennzeichnet, dass das erste Prothesenteil (11) einstückig ist.

32. Gelenkprothese nach einem der Ansprüche 27 bis 30, dadurch gekennzeichnet, dass das Stabilisierungsorgan (14) ein auf ein Basisteil (12) aufgesetztes Teil ist.

33. Gelenkprothese nach einem der Ansprüche 27 bis 32, dadurch gekennzeichnet, dass das zweite Prothesenteil (17) und das Zwischenteil (13) eine Ausnehmung (29, 37) für das hintere Kreuzband aufweisen.

34. Gelenkprothese nach einem der Ansprüche 1 bis 33, dadurch gekennzeichnet, dass die Verbindung von Zwischenteil (13) und Kupplungsorgan (15) derart ist, dass das Kupplungsorgan zusammen mit dem Zwischenteil (13) in bezug auf den zweiten Prothesenteil (17) verschwenkt wird.

## Claims

1. A joint prosthesis, more particularly a knee-joint prosthesis, comprising at least one first prosthesis part (11) comprising an anchoring portion and at least one rotary joint portion (19),
a second prosthesis part (17) comprising at least one anchoring portion (25, 27) and one slicing surface (24),
an intermediate part (13) pivotable round a pivot (33) and comprising at least one rotary joint portion (21) for co-operating with the corresponding rotary joint portion (19) of the first prosthesis part (11) and adapted to move in translation and also to pivot relative to the first prosthesis part (17), and
a coupling means (15) disposed between the second prosthesis part (17) and the intermediate part (13) and
the intermediate part (13) and the coupling means (15) co-operate via a guide part (16) and a guide track (35), not slot-shaped, which receives the guide part and in which the guide part can slide with medial and lateral guidance, and either the guide part (16) or the guide track (35) is disposed on the coupling means (15) and the corresponding counterpart, i.e. the guide track or the guide part, is formed on the intermediate part (13).

2. A joint prosthesis according to claim 1, characterised in that the pivot (33) is disposed on the coupling means (15) and is pivotably mounted in the second prosthesis part (17).

3. A joint prosthesis according to claim 2, characterised in that the pivot (33) is mounted in a bore (31) which extends in an anchoring portion (25).

4. A joint prosthesis according to claim 1, characterised in that the pivot (33') is disposed on the second prosthesis part (17), and the coupling means (15) is mounted for pivoting on the pivot (33').

5. A joint prosthesis according to claim 1, characterised in that the pivot (33) is disposed on the coupling means (15), and the intermediate part (13) is mounted for pivoting on the pivot (33).

6. A joint prosthesis according to claim 1, characterised in that the pivot (33) is mounted on the second prosthesis part (17), and the intermediate part (13) is mounted for pivoting on the pivot.

7. A joint prosthesis according to any of claims 1 to 6, characterised in that the guide track (35) is disposed in the sagittal direction.

8. A joint prosthesis according to claim 7, characterised in that the respective guide track (35) extends in a straight line.

9. A joint prosthesis according to claim 7, characterised in that the guide track (35) extends in an arc with a medial radius of curvature.

10. A joint prosthesis according to any of claims 1 to 9, characterised in that the second part (17) has a sliding surface (24) on which the intermediate part (13) is mounted.

11. A joint prosthesis according to any of claims 1 to 10, characterised in that means (34, 34') are provided for preventing the intermediate part (13) from being lifted off the second prosthesis part (17).

12. A joint prosthesis according to any of claims 1 to 11, characterised in that the mobility of the intermediate part (13) relative to the coupling means (15) is restricted to motion along the guide track (35).

13. A joint prosthesis according to any of claims 1 to 12, characterised in that the guide track (35) and the guide part (16) are in the form of a groove and web, a dovetail, a roof or an arcuate enveloping part.

14. A joint prosthesis according to any of claims 1 to 13, characterised in that the guide track (35) is disposed practically centrally in the intermediate part (13).

15. A joint prosthesis according to any of claims 1 to 14, characterised in that the rotary joint portion of the first prosthesis part is formed by two condyles (19) and that the rotary joint portion of the intermediate part is formed by two corresponding bearing surfaces (21) for co-operating with the condyles (19) of the first prosthesis part (11).

16. A joint prosthesis according to any of claims 1 to 15, characterised in that the rotary joint portion of the first prosthesis part (11) is formed by two condyles (19) and that an intermediate part (13) having a bearing surface (21) and common to both condyles (19) is provided for each of the condyles (19).

17. A joint prosthesis according to claim 16, characterised in that the common intermediate part (13) is guided by two spaced-apart guide tracks (16).

18. A joint prosthesis according to any of claims 1 to 15, characterised in that the rotary joint portion of the first prosthesis part (11) is formed by a condyle (19) and that the rotary joint portion of the intermediate part (13) is formed by a bearing surface (21) for co-operating with the condyle (19) of the first prosthesis part (11).

19. A joint prosthesis according to any of claims 1 to 18, characterised in that the condyle (19) on the first prosthesis part (11) is convex and the associated bearing surface (21) on the intermediate part (13) is correspondingly concave.

20. A joint prosthesis according to any of claims 1 to 18, characterised in that the condyle (19) on the first prosthesis part (11) is concave and the corresponding bearing surface (21) on the intermediate part (13) is correspondingly convex.

21. A joint prosthesis according to any of claims 1 to 20, characterised in that the bearing surface (21) of the intermediate part (13) is formed by an interchangeable bearing element (13') in a recess (45) in the intermediate part.

22. A joint prosthesis according to claim 21, characterised in that the recess (45) extends all the way through.

23. A joint prosthesis according to claim 21, characterised in that the recess (45) does not extend all the way through.

24. A joint prosthesis according to any of claims 1 to 23, characterised in that the intermediate part (13) is mounted so as to be movable on the sliding surface (24).

25. A joint prosthesis according to any of claims 1 to 23, characterised in that the intermediate part (13) is held e.g. by the coupling means (15) at a distance from the sliding surface (24).

26. A joint prosthesis according to any of claims 1 to 25, characterised in that the second part (17) and the intermediate part (13) have a recess (29, 37) for the front and/or rear cruciate ligament.

27. A joint prosthesis according to claim 1 or 2, characterised in that the coupling means (15) has a stabilising element (36) which co-operates with a stabilising means (14) on the first prosthesis part (11) in order to ensure stability even with absent or weak ligament structures.

28. A joint prosthesis according to claim 27, characterised in that the rotary joint portion (19) of the first prosthesis part (11) is cylindrical.

29. A joint prosthesis according to claim 27, characterised in that the rotary joint portion (19) of the first prosthesis part (11) is curved in spherical manner over both condyles.

30. A joint prosthesis according to claim 27, characterised in that the rotary joint portion (19) of the first prosthesis part (11) has two condyle-like spherically curved surfaces (19).

31. A joint prosthesis according to any of claims 27 to 30, characterised in that the first prosthesis part (11) is in one piece.

32. A joint prosthesis according to any of claims 27 to 30, characterised in that the stabilising means (14) is a part mounted on a base part (12).

33. A joint prosthesis according to any of claims 27 to 32, characterised in that the second prosthesis part (17) and the intermediate part (13) have a recess (29, 37) for the rear cruciate ligament.

34. A joint prosthesis according to any of claims 1 to 33, characterised in that the connection of the intermediate part (13) to the coupling means (15) is such that the coupling means together with the intermediate part (13) is pivoted relative to the second prosthesis part (17).

## Revendications

1. Prothèse articulaire, en particulier prothèse du genou, comportant:
au moins une première pièce de prothèse (11), qui présente une partie d'ancrage et au moins une partie d'articulation (19),
une deuxième pièce de prothèse (17), qui présente au moins une partie d'ancrage (25, 27) et une surface de glissement (24),
une pièce intermédiaire (13), qui peut pivoter autour d'un pivot (33) et présente au moins une partie d'articulation (21) destinée à coopérer avec la partie d'articulation correspondante (19) de la première pièce de prothèse (11), et qui peut exécuter aussi bien un mouvement de translation qu'un mouvement de pivotement par rapport à la deuxième pièce de prothèse (17),
un organe d'accouplement (15), qui est disposé entre la deuxième pièce de prothèse (17) et la pièce intermédiaire (13),
la pièce intermédiaire (13) et l'organe d'accouplement (15) coopérant au moyen d'un élément de guidage (16) et d'une coulisse (35) qui n'est pas réalisée sous forme d'un trou oblong, coulisse qui reçoit l'élément de guidage et dans laquelle celui-ci peut glisser en étant guidé dans les directions médiale et latérale, et, soit l'élément de guidage (16), soit la coulisse (35), étant alors disposé sur l'organe d'accouplement (15) et l'élément conjugué correspondant, à savoir la coulisse ou respectivement l'élément de guidage, étant formé sur la pièce intermédiaire (13).

2. Prothèse articulaire selon la revendication 1, caractérisée en ce que le pivot (33) est disposé sur l'organe d'accouplement (15) et est monté pivotant dans la deuxième pièce de prothèse (17).

3. Prothèse articulaire selon la revendication 2, caractérisée en ce que le pivot (33) est monté dans une forure (31), qui s'étend dans une partie d'ancrage (25).

4. Prothèse articulaire selon la revendication 1, caractérisée en ce que le pivot (33') est disposé sur la deuxième pièce de prothèse (17) et en ce que l'organe d'accouplement (15) est monté pivotant sur le pivot (33').

5. Prothèse articulaire selon la revendication 1, caractérisée en ce que le pivot (33) est disposé sur l'organe d'accouplement (15), et en ce que la pièce intermédiaire (13) est montée pivotante sur le pivot (33).

6. Prothèse articulaire selon la revendication 1, caractérisée en ce que le pivot (33) est disposé sur la deuxième pièce de prothèse (17), et en ce que la pièce intermédiaire (13) est montée pivotante sur le pivot.

7. Prothèse articulaire selon l'une des revendications 1 à 6, caractérisée en ce que la coulisse (35) est disposée en direction sagittale.

8. Prothèse articulaire selon la revendication 7, caractérisée en ce que la coulisse respective (35) est rectiligne.

9. Prothèse articulaire selon la revendication 7, caractérisée en ce que la coulisse (35) s'étend selon un arc ayant un rayon de courbure médial.

10. Prothèse articulaire selon l'une des revendications 1 à 9, caractérisée en ce que la deuxième pièce de prothèse (17) présente une surface de glissement (24), sur laquelle la pièce intermédiaire (13) est montée.

11. Prothèse articulaire selon l'une des revendications 1 à 10, caractérisée en ce que des moyens (34, 34') sont prévus pour empêcher un décollement de la pièce intermédiaire (13) par rapport à la deuxième pièce de prothèse (17).

12. Prothèse articulaire selon l'une des revendications 1 à 11, caractérisée en ce que la liberté de mouvement de la pièce intermédiaire (13) par rapport à l'organe d'accouplement (15) est limitée à des mouvements le long de la coulisse (35).

13. Prothèse articulaire selon l'une des revendications 1 à 12, caractérisée en ce que la coulisse (35) et l'élément de guidage (16) sont réalisés sous forme d'une rainure et d'une languette, en queue d'aronde, à recouvrement ou à enveloppement en forme d'arc.

14. Prothèse articulaire selon l'une des revendications 1 à 13, caractérisée en ce que la coulisse (35) est disposée pratiquement en position médiane dans la pièce intermédiaire (13).

15. Prothèse articulaire selon l'une des revendications 1 à 14, caractérisée en ce que la partie d'articulation de la première pièce de prothèse est formée par deux condyles (19) et en ce que la partie d'articulation de la pièce intermédiaire est formée par deux surfaces d'appui correspondantes (21) destinées à coopérer avec les condyles (19) de la première pièce de prothèse (11).

16. Prothèse articulaire selon l'une des revendications 1 à 15, caractérisée en ce que la partie d'articulation de la première pièce de prothèse (11) est formée par deux condyles (19) et en ce qu'une pièce intermédiaire (13), commune pour les deux condyles (19), est pourvue d'une surface d'appui (21) pour chacun des condyles (19).

17. Prothèse articulaire selon la revendication 16, caractérisée en ce que la pièce intermédiaire commune (13) est guidée par deux coulisses (16) disposées à une certaine distance l'une de l'autre.

18. Prothèse articulaire selon l'une des revendications 1 à 15, caractérisée en ce que la partie d'articulation de la première pièce de prothèse (11) est formée par un condyle (19) et en ce que la partie d'articulation de la pièce intermédiaire (13) est formée par une surface d'appui (21) destinée à coopérer avec le condyle (19) de la première pièce de prothèse (11).

19. Prothèse articulaire selon l'une des revendications 1 à 18, caractérisée en ce que chaque condyle (19) de la première pièce de prothèse (11) a une forme convexe et la surface d'appui associée (21) de la pièce intermédiaire (13) a une forme concave correspondante.

20. Prothèse articulaire selon l'une des revendications 1 à 18, caractérisée en ce que chaque condyle (19) de la première pièce de prothèse (11) a une forme concave et la surface d'appui associée (21) de la pièce intermédiaire (13) a une forme convexe correspondante.

21. Prothèse articulaire selon l'une des revendications 1 à 20, caractérisée en ce que chaque surface d'appui (21) de la pièce intermédiaire (13) est formée par un élément d'appui interchangeable (13') placé dans un évidement (45) de cette dernière.

22. Prothèse articulaire selon la revendication 21, caractérisée en ce que l'évidement (45) est continu.

23. Prothèse articulaire selon la revendication 21, caractérisée en ce que l'évidement (45) n'est pas continu.

24. Prothèse articulaire selon l'une des revendications 1 à 23, caractérisée en ce que la pièce intermédiaire (13) est montée glissante sur la surface de glissement (24).

25. Prothèse articulaire selon l'une des revendications 1 à 23, caractérisée en ce que la pièce intermédiaire (13) est maintenue à distance de la surface de glissement (24), par exemple par l'organe d'accouplement (15).

26. Prothèse articulaire selon l'une des revendications 1 à 25, caractérisée en ce que la deuxième pièce de prothèse (17) et la pièce intermédiaire (13) présentent un renfoncement (29, 37) pour le ligament croisé avant et/ou arrière.

27. Prothèse articulaire selon la revendication 1 ou 2, caractérisée en ce que l'organe d'accouplement (15) comporte un élément stabilisateur (36), qui coopère avec un organe stabilisateur (14) de la première pièce de prothèse (11), pour garantir une bonne stabilité même en cas d'absence ou de faiblesse des structures ligamentaires.

28. Prothèse articulaire selon la revendication 27, caractérisée en ce que la partie d'articulation (19) de la première pièce de prothèse (11) est cylindrique.

29. Prothèse articulaire selon la revendication 27, caractérisée en ce que la partie d'articulation (19) de la première pièce de prothèse (11) est bombée, sous une forme sphérique, sur les deux condyles.

30. Prothèse articulaire selon la revendication 27, caractérisée en ce que la partie d'articulation (19) de la première pièce de prothèse (11) présente deux surfaces (19) bombées sous une forme sphérique à la manière d'un condyle.

31. Prothèse articulaire selon l'une des revendications 27 à 30, caractérisée en ce que la première pièce de prothèse (11) est monobloc.

32. Prothèse articulaire selon l'une des revendications 27 à 30, caractérisée en ce que l'organe stabilisateur (14) est un élément rapporté sur un élément de base (12).

33. Prothèse articulaire selon l'une des revendications 27 à 32, caractérisée en ce que la deuxième pièce de prothèse (17) et la pièce intermédiaire (13) présentent un renfoncement (29, 37) pour le ligament croisé arrière.

34. Prothèse articulaire selon l'une des revendications 1 à 33, caractérisée en ce que l'assemblage de la pièce intermédiaire (13) et de l'organe d'accouplement (15) est tel, que l'organe d'accouplement pivote conjointement avec la pièce intermédiaire (13) par rapport à la deuxième pièce de prothèse (17).
